# EUROPEAN PATENT APPLICATION

(11) **EP 4 385 508 A1**
(43) Date of publication of application: **19.06.2024**
(21) Application number: 22306916.2
(22) Date of filing: 16.12.2022
(51) Int. Cl.: A61K 31/341, A61P 21/00, A23L 29/00

(54) **FURAN FATTY ACIDS FOR ENHANCING MUSCLE MASS**

(71) Applicant: Centre de Coopération Internationale en Recherche Agronomique pour le Développement (CIRAD), 75116 Paris (FR); Kasetsart University, Bangkok 10900 (TH); Institut national de recherche pour l'agriculture, l'alimentation et l'environnement (INRAE), 75007 Paris (FR)
(72) Inventor: CASAS, François, 34980 Saint-Gély-du-Fesc (FR); FEILLET-COUDRAY, Christine, 34090 MONTPELLIER (FR); LIENGPRAYOON, Siriluck, 10900 BANGKOK (TH); DURAND, Erwann, 34170 Castelnau-le-Lez (FR); VAYSSE, Laurent, 34000 MONTPELLIER (FR)
(74) Representative: Cabinet Nony

(57) **Abstract**

The present disclosure relates to a furan fatty acid, or a furan fatty acid-containing compound, for its use for preventing and/or treating a loss in muscle mass in a human or animal subject. It also pertains to a non-therapeutic use of a furan fatty acid, or of a furan fatty acid-containing compound for maintaining or increasing muscle mass in a subject in need thereof, in particular in a subject selected from a malnourished subject, an elderly subject, in particular a malnourished elderly subject, and a subject engaged in strenuous exercise.

## Description

### FIELD OF THE DISCLOSURE

The present disclosure relates to the field of medical and non-medical needs for increasing muscle mass and/or muscle function.

### BACKGROUND OF THE DISCLOSURE

The proportion of elderly people (65 years and older) is expected to represent one third of the French population by 2050. This population is very heterogeneous and is characterized, at the extremes, by individuals in good health, who are active and in good nutritional condition, with little sarcopenia (age-related loss of mass and muscle function) and generally living at home. Conversely, there is (at the same age) an elderly population that is undernourished and highly sarcopenic, generally dependent and living in institutions and who are in a state of chronic undernutrition (Buckinx et al., Burden of frailty in the elderly population: perspectives for a public health challenge. Archives of public health = Archives belges de santé publique. 2015;73(1): 19). At the same time, in some Western countries, overweight or obese individuals represent more than half of the population. In addition to an increased susceptibility to diabetes or cardiovascular pathologies, part of this population may also present increased muscle wasting which may reduce the autonomy of these individuals and, above all, limit the room for treatment flexibility when treating obesity with low-calorie diets (Barazzoni et al., Sarcopenic Obesity: Time to Meet the Challenge. Obesity facts. 2018;11(4):294-305). In addition, loss of muscle mass and function can also punctually affect individuals over the course of their lives, depending on their background and experiences. This is particularly the case for "burn victims", immobilized people, cancer patients or people who have undergone intestinal resection or who have intestinal malabsorptions.

Presently, enhancing muscle mass or muscle function may be obtained at least by practicing physical exercise. Indeed, practicing physical exercise cannot be performed by subjects such as immobilized subjects (*e.g.* subjects confined to bed following a bone fracture) or the elderly affected with some physical weakness.

A need for enhancing muscle mass also exists in subjects affected with sarcopenia in pathological situations, such as in cancer subjects affected with severe sarcopenia. How to better prevent sarcopenia in these subjects prevent and treat sarcopenia has become one of the frontiers of global geriatric research. Many molecules have been identified as therapeutic targets, bringing a booming of drug investigation targeting sarcopenia. It may be cited the exploration of pharmacotherapies designed or repurposed for treating sarcopenia, such as (i) drugs targeting myostatin signalling, including myostatin inhibitors, activin receptor antagonists, follistatin fusion proteins and gene therapy, (ii) drugs targeting the renin-angiotensin system, such as ACE inhibitors, angiotensin II type I receptor antagonists, Mitochondrial Assembly Receptor (MASR) agonists, (iii) Testosterone and selective androgen receptor modulators, such as testosterone and (iv) Ghrelin and its mimetics.

There remains a need for further substances or compositions able to enhance muscle mass or enhance muscle function both (i) for providing to healthy subjects in which preventing sarcopenia or increasing muscle mass or muscle function is desirable and (ii) for preventing or treating sarcopenia in subjects affected with a disease.

### SUMMARY OF THE DISCLOSURE

The present disclosure relates to a furan fatty acid, or a furan fatty acid-containing compound, for its use for preventing and/or treating a loss in muscle mass in a human or animal subject.

In preferred embodiments, the furan fatty acid, or a furan fatty acid-containing compound, for its use according to claim 1, wherein the furan fatty acid or the furan fatty acid-containing compound is of formula (I) : 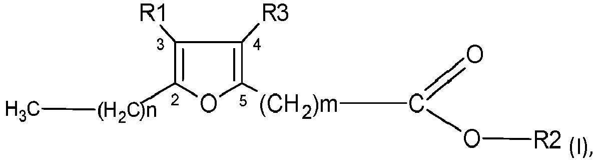 wherein
(i) m is an integer ranging from 1 to 12 ,
(ii) n is an integer ranging from 1 to 12,
(iii) R1and R3 represent, independently of each other, a hydrogen atom, a methyl group or a carboxyl group, and
(iv) R2 means a hydrogen atom, a (C1-C22) alkyl group, a (C2-C22) alkenyl group, a (C2-C22) alkynyl group or a group of formula (A1) or (A2) : 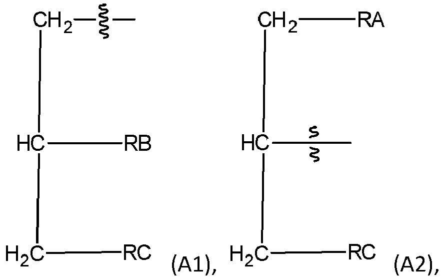 wherein 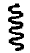
   - symbol« » in formula (A1) means the attachment site between the CH₂ group thereof and the oxygen atom of the carboxyl group of the compound of formula (I), 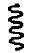
   - symbol« » in formula (A2) means the attachment site between the CH group thereof and the oxygen atom of the carboxyl group of the compound of formula (I), and
   - RA, RB and RC represent, independently of each other,
   - a hydroxyl group, or
   - -O-C(=O)-R4 , wherein R4 means :
      - a group of formula (III) : 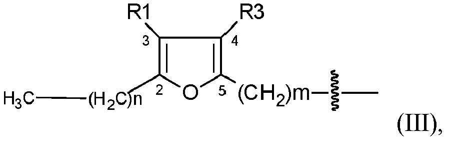 (III), wherein R1, R3, 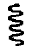 m and n are as defined for compound of formula (I) and symbol« » in formula (III) means the attachment site between the (CH₂)m thereof and the carbonyl group of the -O-C(=O)-R4, or
      - a (C6-C22) linear or branched, alkyl group, a (C6-C22) linear or branched alkenyl group or a (C6-C22) alkynyl group.

In some embodiments of a furan fatty acid or furan fatty acid-containing compound above, for its use according to the present disclosure,
- each of R1, R3, m and n, are as defined for formula (I),
- R2 means a group of formula (A1) wherein RB and RC are as defined in claim 2 with at least one of RB and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH2)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂
- R2 means a group of formula (A2) wherein RA and RC are as defined in claim 2 with at least one of RA and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH₂)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂.

In preferred embodiments, a furan fatty acid of formula (I) as used herein is selected from the furan fatty acids wherein,
- n is 2, m is 8, R1 means a methyl group, R2 means a hydrogen atom and R3 means a methyl group, or
- n is 4, m is 8, R1 means a hydrogen atom, R2 means a hydrogen atom and R3 means a methyl group, or
- n is 4, m is 8, R1 means a methyl group, R2 means a hydrogen atom and R3 means a methyl group, or
- n is 2, m is 10, R1 means a methyl group, R2 means a hydrogen atom and R3 means a methyl group, or
- n is 4, m is 10, R1 means a hydrogen atom, R2 means a hydrogen atom and R3 means a methyl group, or
- n is 4, m is 10, R1 means a methyl group, R2 means a hydrogen atom and R3 means a methyl group, or
- n is 4, m is 12, R1 means a hydrogen atom, R2 means a hydrogen atom and R3 means a methyl group , or
- n is 4, m is 12, R1 means a methyl group, R2 means a hydrogen atom and R3 means a methyl group.

In preferred embodiments, a furan fatty acid-containing compound as used herein is of formula (II) below 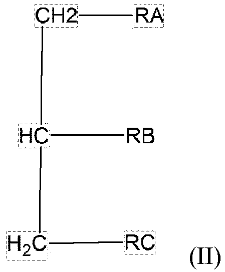 wherein groups RA, RB and RC represent, independently of each other, (i) a hydroxyl group, (ii) -O-C(=O)-R4, wherein R4 means a group of formula (III) as defined above or (iii)-O-C(=O)-R4, wherein R4 means a (C6-C22) linear or branched, alkyl chain, a (C6-C22) linear or branched alkenyl group or a (C6-C22) linear or branched alkynyl group provided that at least one of RA, RB and RC represents -O-C(=O)-R4 , wherein R4 means a group of formula (III) as defined above.

In preferred embodiments of a furan fatty acid-containing compound of formula (II) above, RA, RB and RC are identical and each means -O-C(=O)-R4 , wherein R4 means a group of formula (III) as defined in claim 2 wherein n is 4, m is 8, R1 means a hydrogen atom and R3 means a methyl group.

Preferably, a subject according to the present disclosure is a human subject or an animal subject.

In some embodiments of the uses of a furan fatty acid or of a furan fatty acid-containing compound disclosed herein, the human or animal subject is selected among elderly subjects affected by sarcopenia, overweight and obese subjects affected by sarcopenic obesity and subjected to a food regime, subjects affected by cachexia, notably a cachexia linked to cancer, subjects affected by an inflammatory bowel disease or a chronic obstructive pulmonary disease, immobilized subjects, subjects with severe burns and affected with septic or viral infections such as with a SARS-CoV-2 virus, subjects having a heart failure or a respiratory failure, subjects having cirrhosis, subjects affected with a rheumatoid arthritis" convalescent subjects, immobilized subjects, and subjects with bowel resections or intestinal malabsorptions.

In some embodiments, a furan fatty acid, or a furan fatty acid-containing compound, for any use according to the present disclosure, is comprised in an oral composition, and more particularly in an oral composition selected from the group consisting of a food product, a beverage, a pharmaceutical, a nutraceutical, a food additive, a dietary supplement, a dairy product and a living biotherapy product (LBPs).

The present disclosure also pertains to the non-therapeutic use according to claim 8, wherein the furan fatty acid, or the furan fatty acid-containing compound, especially the furan fatty acid, or the furan fatty acid-containing compound of formula (I) or (II) as described herein, for maintaining or increasing muscle mass in a subject in need thereof, in particular in a subject selected from a malnourished subject, an elderly subject, in particular a malnourished elderly subject, and a subject engaged in strenuous exercise.

### DESCRIPTION OF THE FIGURES

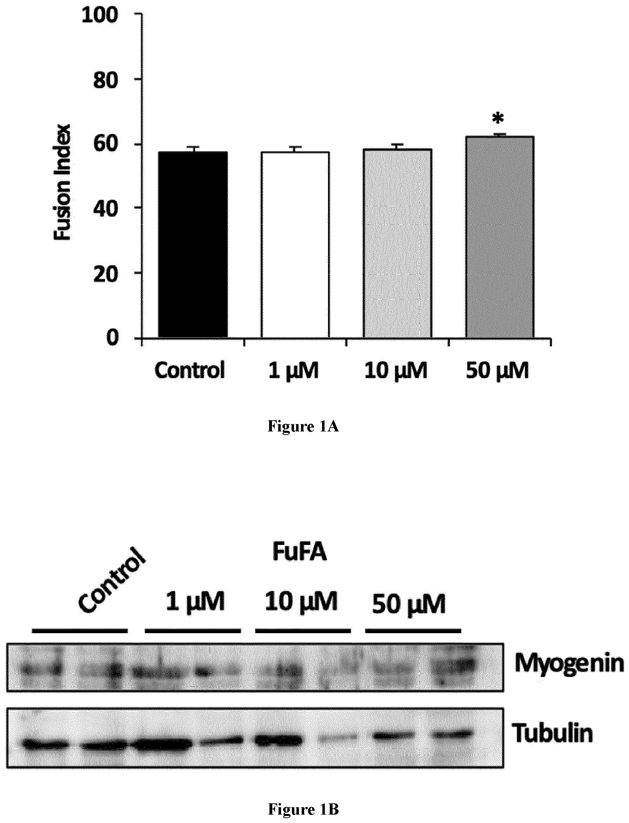

**Figure 1** illustrates FuFA-F2 supplementation had little influence on C2C12 myoblast differentiation. Figure 1A : The extent of differentiation of C2C12 myoblasts was assessed by the fusion index (percentage of nuclei incorporated into myotubes relative to the total number of nuclei). Figure 1 B : Myogenin protein levels were analyzed by Western blot (n = 6 for each group). Typical blots are shown. Proteins were quantified with Image Lab^{™} Touch Software. Statistical Significance: *p<0.05 vs Control, Student's *t*-test. Results are expressed as mean ± SEM.

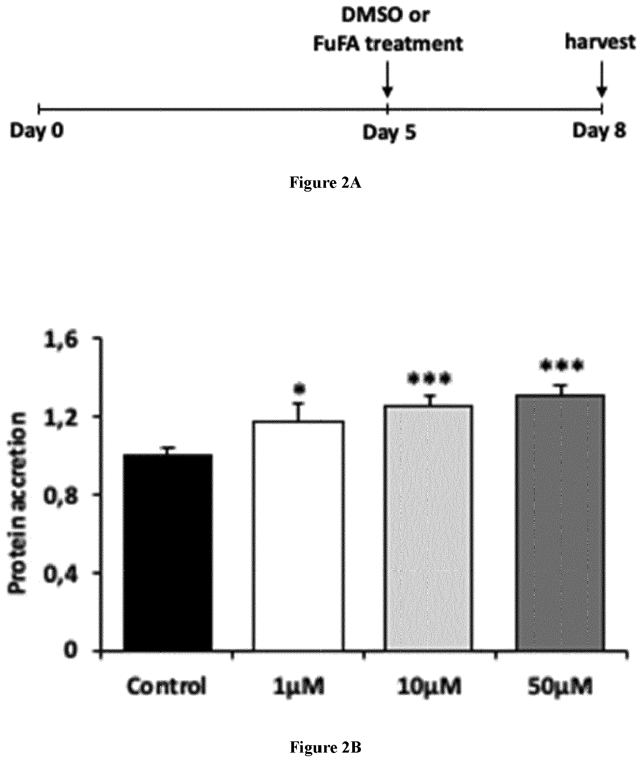

**Figure 2** Illustrates FuFA-F2 supplementation increases protein accretion in C2C12 myotubes. Figure 2A : Scheme of the experiment. Figure 2B : Relative protein content (n=6 for each group). Statistical Significance: *p<0.05; ***p<0.001 vs Control, Student's *t*-test. Results are expressed as mean ± SEM.

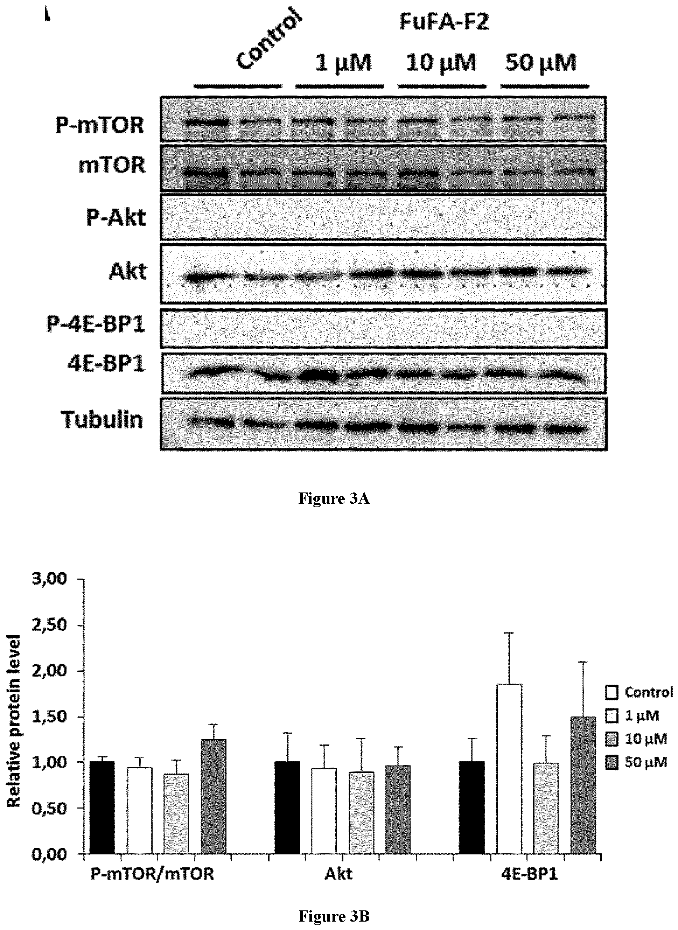

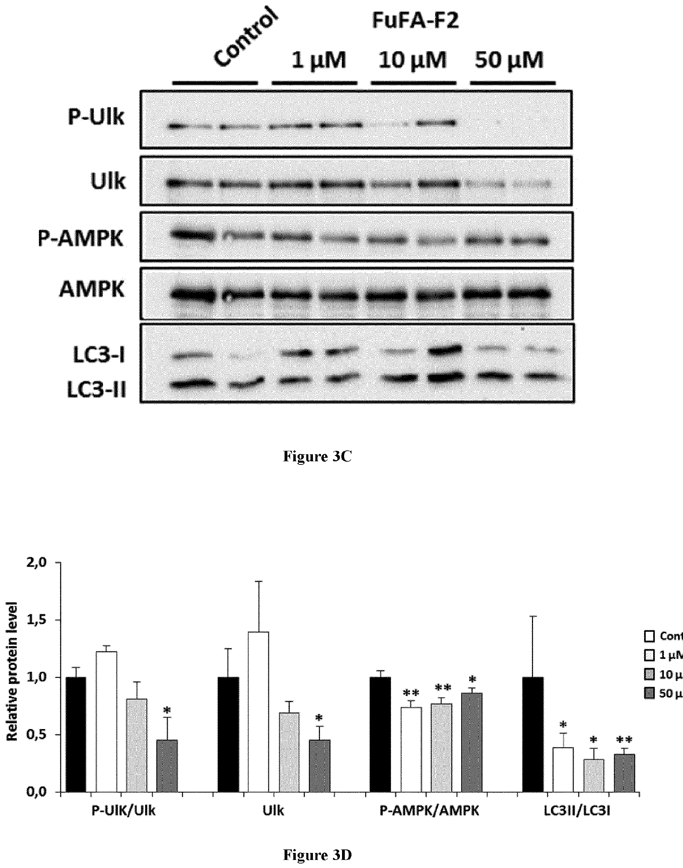

**Figure 3** Illustrates FuA-F2 supplementation decreases autophagy in C2C12 myotubes. Figure 3A : Western-blot analysis of the indicated proteins (*n* = 6 for each group). Typical blots are shown. Figure 3B : Quantification of relative protein expression in C2C12 myotubes. Figure 3C : Western-blot analysis of the indicated proteins (*n* = 6 for each group). Typical blots are shown. Figure 3D : Quantification of relative protein expression in C2C12 myotubes. Proteins were quantified with Image Lab^{™} Touch Software. Statistical Significance: *p<0.05; **p<0.01 vs Control, Student's *t*-test. Results are expressed as mean ± SEM.

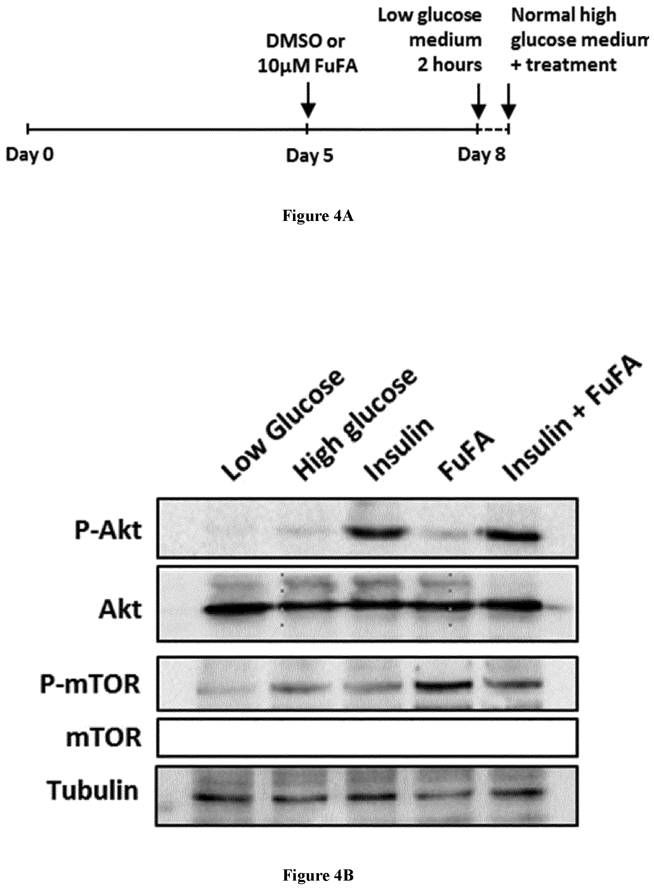

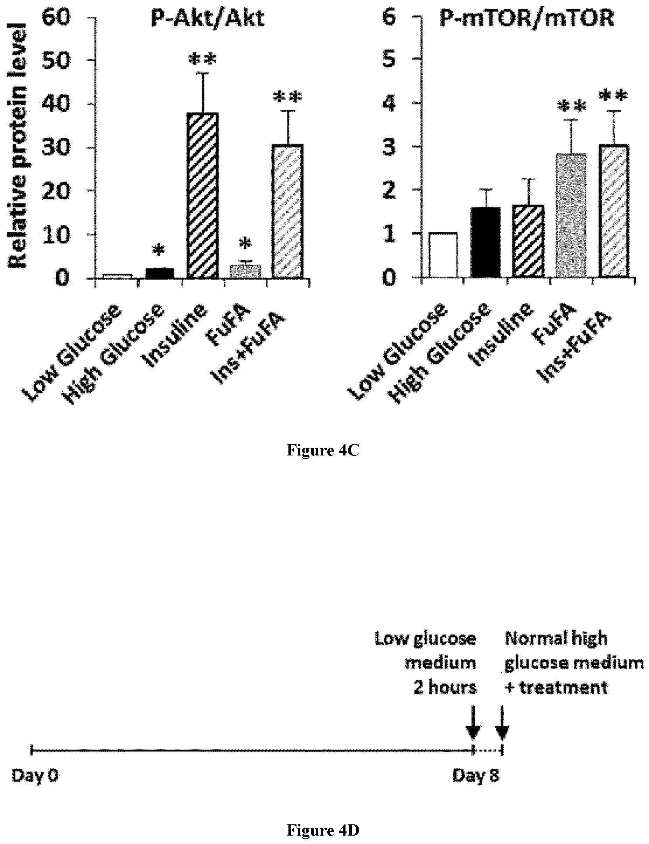

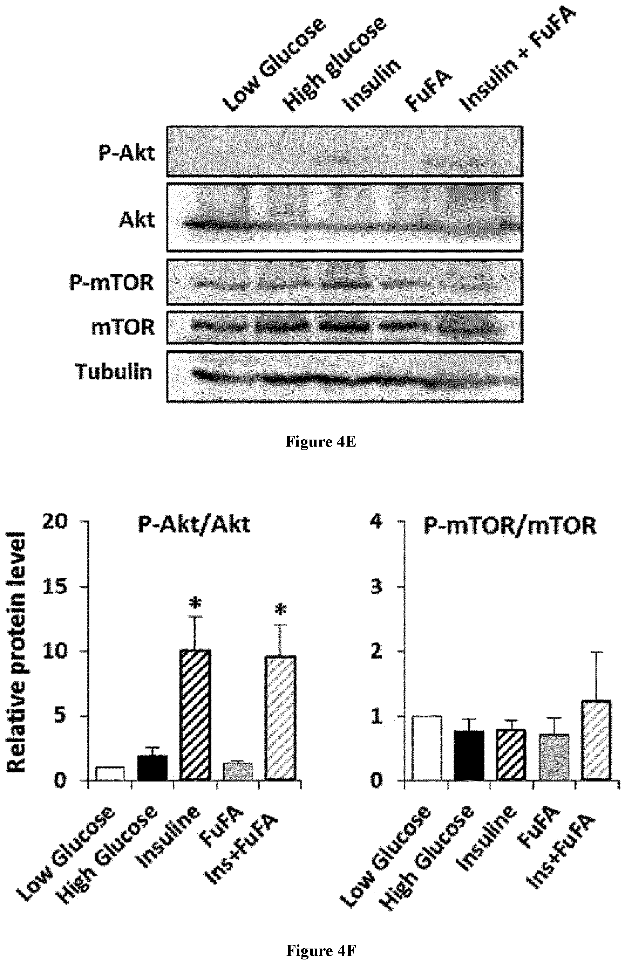

**Figure 4** Illustrates FuFA-F2 supplementation increase mTOR phosphorylation in an Akt independent manner. Figure 4A : Scheme of the experiment. Figure 4B : Western-blot analysis of the indicated proteins (*n* = 6 for each group). Typical blots are shown. Figure 4C : Quantification of relative protein expression in C2C12 myotubes. Figure 4D : Scheme of the experiment. Figure 4E : Western-blot analysis of the indicated proteins (*n* = 6 for each group). Typical blots are shown. Figure 4F : Quantification of relative protein expression in C2C12 myotubes. Statistical Significance: *p<0.05; **p<0.01 vs Control, Student's *t*-test. Results are expressed as mean ± SEM.

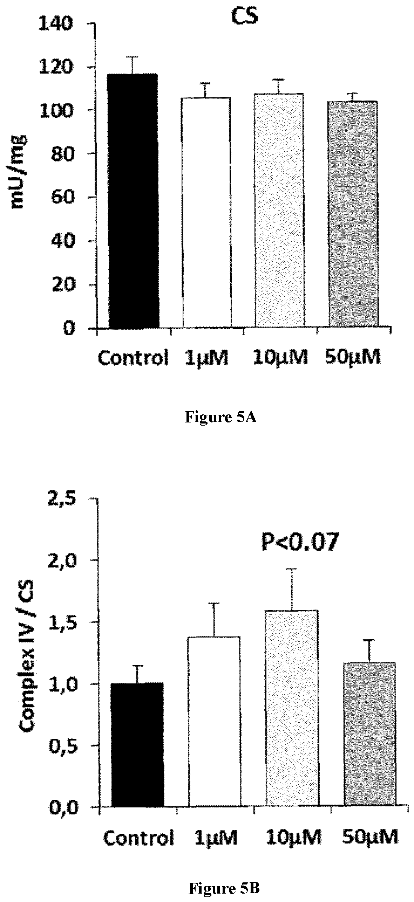

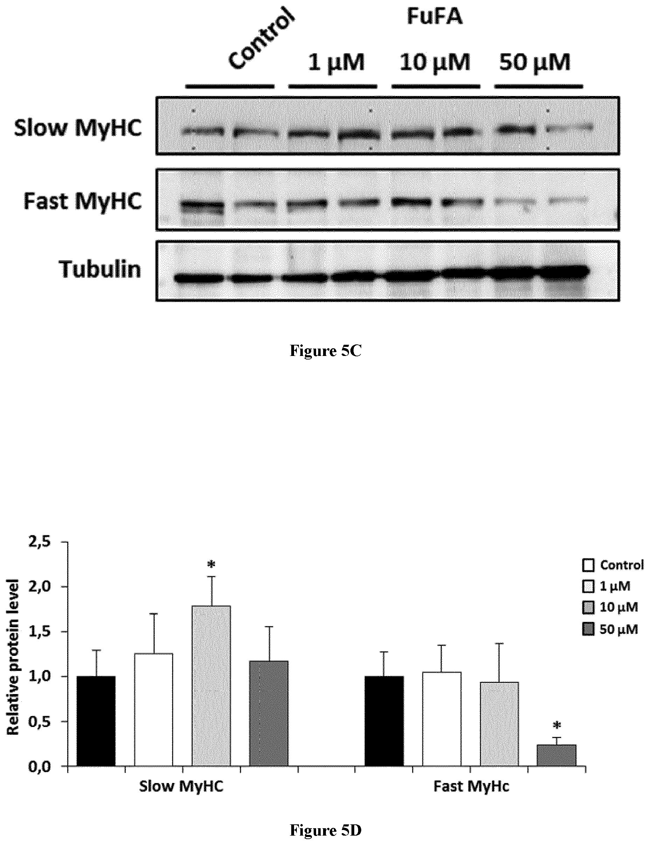

**Figure 5** Illustrates FuFA-F2 supplementation modulates the metabolic and contractile characteristics of muscle fibers. Figure 5A : Citrate synthase activity of C2C12 myotubes. Enzymatic activity was measured 3 days after FuFA-F2 treatment at the indicated concentration (n = 6 for each group). Figure 5B : Complex IV activity of C2C12 myotubes. Enzymatic activity was measured 3 days after FuFA-F2 treatment at the indicated concentration (n = 6 for each group). Figure 5C : Slow and Fast MyHC protein levels were analyzed by Western blot (*n* = 6 for each group). Typical blots are shown. Figure 5D : Quantification of relative MyHC expression in C2C12 myotubes. Proteins were quantified with Image Lab^{™} Touch Software. Statistical Significance: *p<0.05 vs Control, Student's *t*-test. Results are expressed as mean ± SEM.

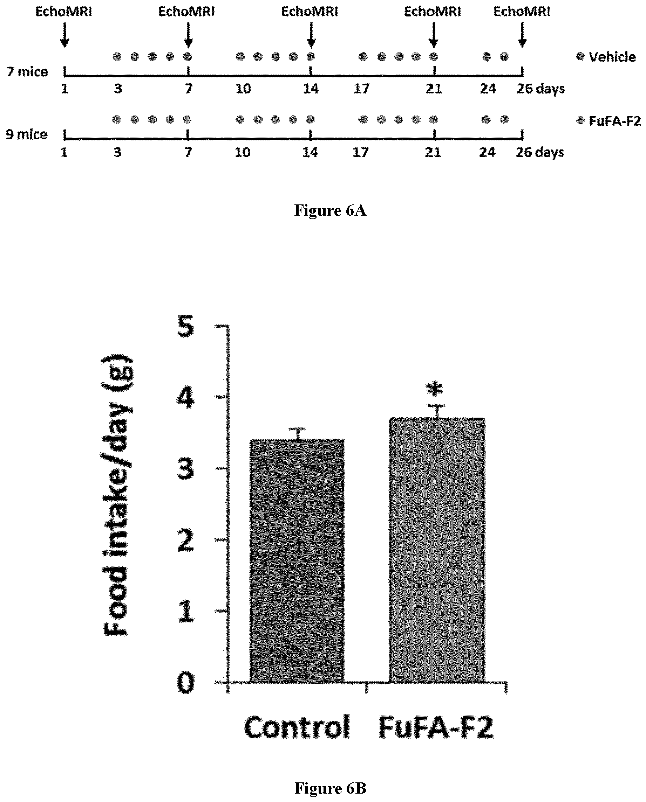

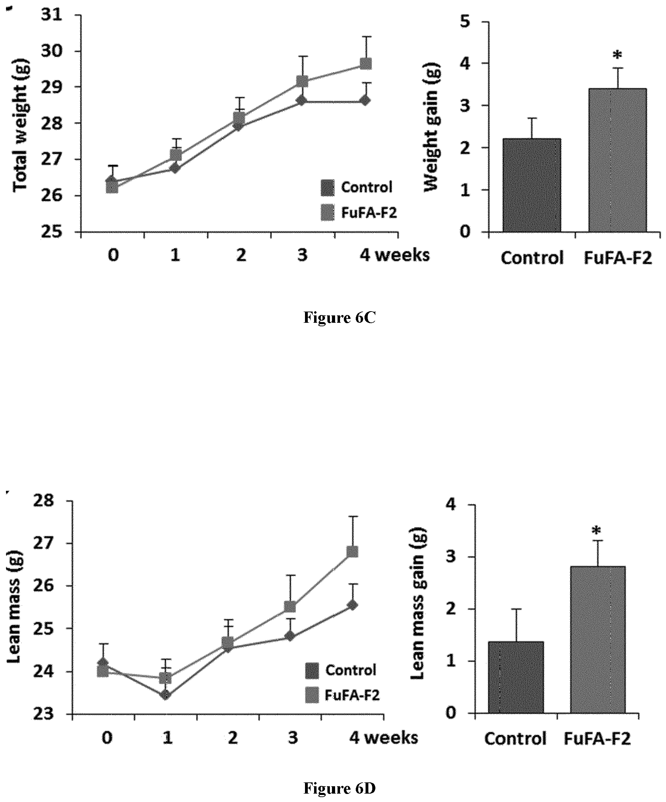

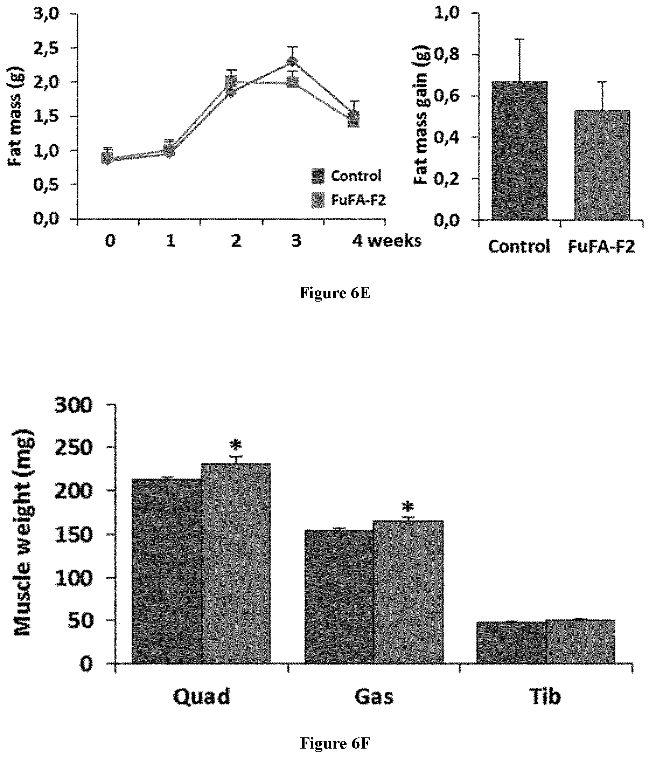

**Figure 6** Illustrates FuFA-F2 supplementation in C57Bl6 mice increases muscle mass. Figure 6A : Design of the study : Upper line : Vehicle; lower line : FuFA-F2. Figure 6B : Food intake. Figure 6C : Total weight and weight gain. Higher curve with "■" : FuFA-F2; Lower curve with "+" : Control. Figure 6D : Lean mass and lean mass gain. Higher curve with "■" : FuFA-F2; Lower curve with "1" : Control. Figure 6E : Fat mass and fat mass gain. Curve with "■" : FuFA-F2; Curve with "+" : Control Figure 6F : Quadriceps, gastrocnemius, and tibialis muscles weight. Left bars : Control; Right bars : FuFA-F2. Statistical Significance: *p<0.05 vs Control, Student's *t*-test. Results are expressed as mean ± SEM. n = 7 for control and n = 9 for FuFA-F2.

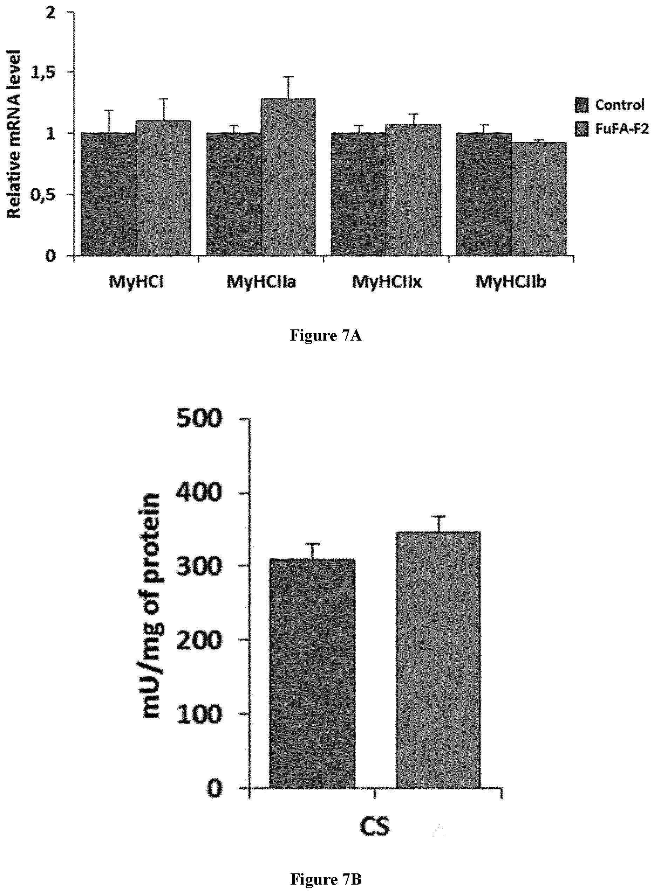

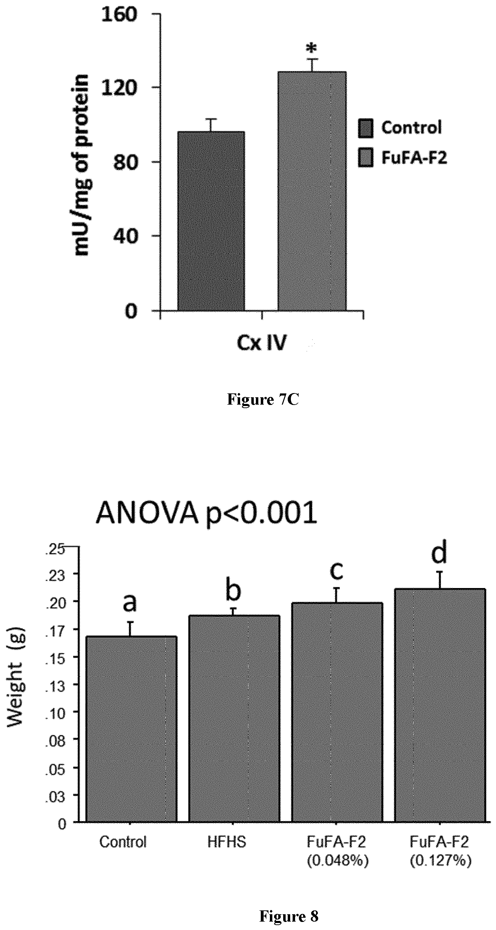

**Figure 7** Illustrates FuFA-F2 supplementation in C57Bl6 mice increases mitochondrial complex IV activity in quadriceps muscle. Figure 7A : Relative expression of myosin heavy chains. Left bars : Control; Right bars : FuFA-F2. Figure 7B : Citrate synthase activity. Left bar : Control; Right bar : FuFA-F2. Figure 7C : Mitochondrial complex IV activity.. Left bar: Control; Right bar : FuFA-F2.Statistical Significance: *p<0.05 vs Control, Student's *t*-test. Results are expressed as mean ± SEM. n = 7 for control and n = 9 for FuFA-F2.
**Figure 8** Illustrates the *in vivo* dose-dependent effect of FuFA-F2 incorporated in a High Fat and High Sucrose (HFHS) diet on muscle mass. Ordinate : variation of the quadriceps muscle mass at the end of the 3-month time period of feeding with the specified nutritional compositions (in grams). Abscissa : each bar represents the muscle mass results in the following animal groups, from left to right: (i) Control group fed with a standard nutritional formula without added FuFA-F2, (ii) Animals fed with a High Fat and High Sucrose (HFHS) diet, (iii) Animals fed with a High Fat and High Sucrose (HFHS) diet added with 0.048% of FuFA-F2by weight, based on the total weight of the composition and (iv) Animals fed with a High Fat and High Sucrose (HFHS) diet added with 0.217% of FuFA-F2 by weight, based on the total weight of the composition.

### DETAILED DESCRIPTION

### Definitions

The terms used in this specification generally have their ordinary meanings in the art. Certain terms are discussed below, or elsewhere in the present disclosure, to provide additional guidance in describing the products and methods of the presently disclosed subject matter.

The following definitions apply in the context of the present disclosure:
As used in this specification and the appended claims, the singular forms "a", "an" and "the" include plural referents unless the content clearly dictates otherwise.

The term "about" as used herein refers to the usual error range for the respective value readily known to the skilled person in this technical field. Reference to "about" a value or parameter herein includes (and describes) embodiments that are directed to that value or parameter *per se.* In some embodiments, the term "about" refers to ±10% of a given value. However, whenever the value in question refers to an indivisible object, such as a nucleotide or other object that would lose its identity once subdivided, then "about" refers to ±1 of the indivisible object.

It is understood that aspects and embodiments of the present disclosure described herein include "having," "comprising," "consisting of," and "consisting essentially of' aspects and embodiments. The words "have" and "comprise," or variations such as "has," "having," "comprises," or "comprising," will be understood to imply the inclusion of the stated element(s) (such as a composition of matter or a method step) but not the exclusion of any other elements. The term "consisting of' implies the inclusion of the stated element(s), to the exclusion of any additional elements. The term "consisting essentially of' implies the inclusion of the stated elements, and possibly other element(s) where the other element(s) do not materially affect the basic and novel characteristic(s) of the invention.

As used herein, a "(Cx-Cy)alkyl" group is a linear or branched saturated hydrocarbon-based aliphatic group comprising from x to y carbon atoms, for example from 1 to 12 carbon atoms, or from 6 to 22 carbon atoms. By way of examples, mention may be made of, but not limited to: methyl, ethyl, propyl, n-propyl, isopropyl, butyl, isobutyl, sec-butyl, tert-butyl, pentyl, isopentyl, hexyl, isohexyl, octyl, nonyl, decyl groups, and the like;

As used herein a "(Cx-Cy)alkenyl" group is a linear or branched hydrocarbon-based aliphatic group comprising at least one unsaturation (double bond) and comprising, from x to y carbon atoms (x being an integer of at least 2), for example from 2 to 22 carbon atoms. By way of examples, mention may be made of, but not limited to: ethenyl, propenyl, butenyl, pentenyl, hexenyl groups, and the like;

As used herein, a "(Cx-Cy)alkynyl" goup: a linear or branched hydrocarbon-based aliphatic group comprising at least one triple bond and comprising from x to y carbon atoms (x being an integer of at least 2) for example from 2 to 22 carbon atoms. By way of examples, mention may be made of, but not limited to: ethynyl, propynyl, butynyl, pentynyl, hexynyl groups, and the like;

### Description

Unexpectedly, the present inventors have found that furan fatty acids, which may be also termed "FuFAs" herein, have the ability to increase protein content in myoblast cells, especially in myoblast-containing myotubes. Notably, the inventors have found that FuFAs decrease autophagy in myoblasts, by inhibiting autophagosomes formation. The inventors have also shown that administration of various FuFAs as well as FuFA-containing compounds increases muscle mass and promotes muscle oxidative metabolism.

Altogether, the inventors' findings demonstrate that FuFAs stimulate muscle anabolism and thus are useful for enhancing muscle mass and/or muscle function in a variety of subjects in need thereof.

Consequently, the present disclosure relates to the use (i) of a furan fatty acid, (ii) of a furan fatty acid-containing compound and (iii) of a composition comprising a furan fatty acid and/or a furan fatty acid-containing compound, wherein furan fatty acids and furan fatty acid-containing compounds are as described herein, for (a) preventing a loss in muscle mass, (b) treating a loss in muscle mass or (c) increasing muscle mass, in a human subject or in an animal subject (*i.e.* in a non-human animal subject). As it will appear clearly in the present disclosure, the said use encompasses embodiments which are therapeutic uses and embodiments which are non-therapeutic uses.

The present disclosure relates to a furan fatty acid, or a furan fatty acid-containing compound, for its use for preventing and/or treating a loss in muscle mass and/or a loss in muscle function in a human or an animal subject.

The present disclosure relates to the use of a furan fatty acid, or a furan fatty acid-containing compound, for manufacturing a composition for preventing or treating a loss in muscle mass and/or a loss in muscle function in a human or an animal subject.

The present disclosure relates to a method for preventing or treating a loss in muscle mass and/or a loss in muscle function in a human or in an animal subject comprising a step of administering to the said human or animal subj ect a furan fatty acid, or a furan fatty acid-containing compound.

The present disclosure further pertains to the use of a furan fatty acid, or of a furan fatty acid-containing compound for increasing muscle mass in a human or an animal subject.

This disclosure also concerns a method for increasing muscle mass in a human or an animal subject comprising administering the said human or animal subject with a furan fatty acid or with a furan fatty acid-containing compound.

In some embodiments, increasing muscle mass concern human or animal subjects wherein loss of muscle mass has not occurred, or is not likely to occur, which encompasses human or animal subjects which are not affected, or likely to be affected, by any physiological state, including any disease, causing a loss in muscle mass. According to these embodiments, increasing muscle mass concern healthy human or animal subjects.

As used herein, the expression "a furan fatty acid" encompasses "one or more furan fatty acids".

As used herein the expression "a furan fatty acid-containing compound, encompasses "one or more furan fatty acid-containing compounds".

Furan fatty acids, are a group of fatty acids comprising a furan ring (i) wherein the first carbon atom in alpha position from the oxygen atom (in position 5 of the furan ring) is substituted by an unbranched alkyl-carboxylic acid group, (ii) wherein the other (second) carbon atom in alpha position from the oxygen atom (in position 2 of the furan ring) is substituted by an alkyl group and (iii) wherein the furan ring is further either non-methylated, mono-methylated or dimethylated on the remaining carbon atoms in positions 3 and 4, respectively.

Furan fatty acids may be contained in furan fatty acid -containing compounds, such as mono-, di- and tri-acylglycerols wherein one, two or the three hydroxyl groups are esterified with a furan fatty acid.

Illustratively, a plurality of furan fatty acids that can be purified from natural sources, such as from a latex of *Hevea brasiliensis,* where they are initially present mainly in the form of triglycerides of furan fatty acids.

Thus, preferably herein, a furan fatty acid or a furan fatty acid-containing compound is of the formula (I) below: 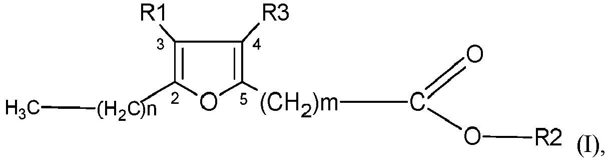 wherein
(i) m is an integer ranging from 1 to 12 ,
(ii) n is an integer ranging from 1 to 12,
(iii) each of R1 and R3, identical or distinct one from the other, means a hydrogen atom, a methyl group or a carboxyl group, and
(iv) R2 means a hydrogen atom, a (C1-C22) alkyl group, a (C2-C22) alkenyl group, a (C2-C22) alkynyl group or a group of formula (A1) or of formula (A2) : 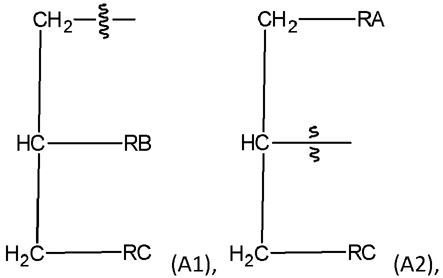 wherein 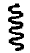
   - symbol« » in formula (A1) means the attachment site between the CH₂ group thereof and the oxygen atom of the carboxyl group of the compound of formula (I), 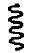
   - symbol« » in formula (A2) means the attachment site between the CH group thereof and the oxygen atom of the carboxyl group of the compound of formula (I), and
   - RA, RB and RC represent, independently of each other,
   - a hydroxyl group, or
   - -O-C(=O)-R4 , wherein R4 means :
      - a group of formula (III) : 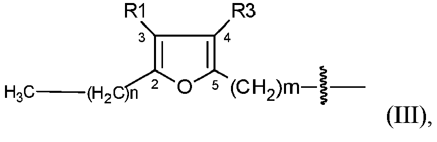 (III), wherein R1, R3, 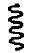 m and n are as defined for compound of formula (I) and symbol« » in formula (III) means the attachment site between the (CH₂)m thereof and the carbonyl group of the -O-C(=O)-R4, or
         or
      - a (C6-C22) linear or branched, alkyl group, a (C6-C22) linear or branched alkenyl group or a (C6-C22) linear or branched alkynyl group.

In preferred embodiments of a furan fatty acid or of a furan fatty acid-containing compound of formula (I), for any use according to the present disclosure,
- each of R1, R3, m and n, are as defined for formula (I),
- R2 means a group of formula (A1) wherein RB and RC are as defined in claim 2 with at least one of RB and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH2)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂, or
- R2 means a group of formula (A2) wherein RA and RC are as defined in claim 2 with at least one of RA and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH2)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂.

In preferred embodiments of a compound of formula (I), m is an integer ranging from 2 to 4.

In preferred embodiments of a compound of formula (I), n is an integer ranging from 8 to 12.

In other preferred embodiments of a compound of formula (I), m is an integer ranging from 2 to 4 and n is an integer ranging from 8 to 12.

In preferred embodiments, R2 is selected from a hydrogen atom and a (C1-C22) alkyl group, and especially a (C1-C12) alkyl group. In more preferred embodiments, R2 is a hydrogen atom.

In other preferred embodiments, R2 is a group of formula (A1) with RB, RC being as defined above.

In further preferred embodiments, R2 is a group of formula (A2) with RA and RC being as defined above.

In preferred embodiment, the furan fatty acid-containing compound is of formula (I) with R1, R3, m and n being as described for formula (I) and wherein R2 is a group of formula (A1) wherein RB and RC represent, independently of each other, - -O-C(=O)-R4 , wherein R4 means a group of formula (III) : 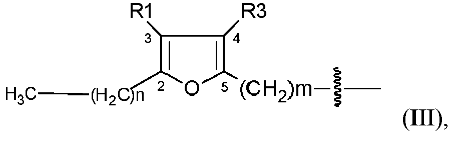 (III), wherein R1, R3, m and 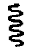 n are as defined for compound of formula (I) and symbol« » in formula (III) means the attachment site between the (CH₂)m thereof and the carbonyl group of the -O-C(=O)-R4,

These preferred embodiments encompass furan fatty acid-containing compounds wherein each furan fatty acid groups comprised therein are identical.

In some of these preferred embodiments of a furan fatty acid-containing compound,
- in the compound of formula (I), n is 2, m is 8, R1 means a methyl group, R3 means a methyl group and R2 means a group of formula (A1), wherein RB and RC are identical and mean O-C(=O)-R4 , wherein R4 means : a group of formula (III) : 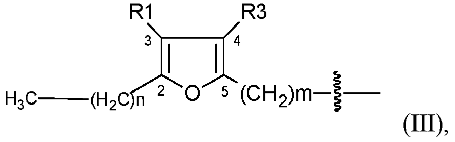 (III), wherein n is 2, m is 8, R1 means a methyl group and R3 means a methyl group.

In some other of these preferred embodiments, embodiments of a furan fatty acid-containing compound,
- in the compound of formula (I), n is 4, m is 8, R1 means a hydrogen atom, R3 means a methyl group and R2 means a group of formula (A1), wherein RB and RC are identical and mean O-C(=O)-R4 , wherein R4 means : a group of formula (III) : 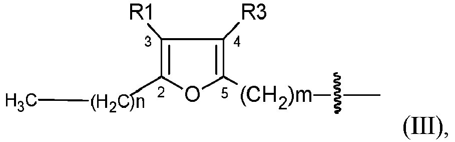 (III), wherein n is 4, m is 8, R1 means a methyl group and R3 means a methyl group.

As it is well known in the art, furan fatty acids (also termed "FuFAs" herein) are fatty acids present in small quantities which have a furan ring (Xu et al., 2017, Prog Lipid Res, Vol. 68 : 119-137). FuFAs are synthesized in plants (Batna et al., 1993, Biochim Biophys Acta, Vol. 1166(2-3) : 171-176) and microorganisms (Lemke et al., 2020, J Biol Chem, Vol. 295(29) : 9786-9801; Shirasaka et al., 1997, Biochim Biophys Acta, Vol. 1346(3) : 253-260). Through food, FuFAs are also found in animals and humans. Fish is a particularly important source of FuFAs, due to its high consumption of algae and marine microorganisms rich in FuFAs (Hannemann et al., 1989, Lipids, Vol. 24(4): 296-298). In commercial fish oils, up to 1% of the total fatty acids can be found in the form of FuFAs (Scrimgeour et al., 1977, J Am Oil Chem Soc, Vol. 54(5) : 210-211). Interestingly, these fatty acids are particularly abundant in certain clones of *Hevea brasiliensis* latex used to produce latex (Hasma et al., 1978, Lipids, Vol. 13(12) : 905-907; Liengprayoon et al., 2011, Phytochemistry, Vol. 72(14-15) : 1902-1913). Furan fatty acids can also be prepared by chemical synthesis, such as disclosed in US 2012/0302774. Further, esters of furan fatty acids can be prepared according to methods well known in the art, such as described, for example, by Bantchev et al. (2014, J American Oil Chemists' Society, Vol. 9 (12) : 2117-2123).

Various biological effects of FuFAs have been described in the art. However, only a few studies have been performed to test biological function of FuFAs. *In vitro,* in 3T3-L1 adipocyte cells, FuFAs increase the expression of adiponectin, a protein known for its anti-inflammatory and insulinosensitive effects (Lauvai et al., 2019, Lipids, Vol. 54(5) : 277-288). Further, *in vivo,* in a rat model of arthritis, FuFAs have a greater anti-inflammatory activity than eicosapentaenoic acid (EPA) (Waksimoto et al., 2011, Proc Nat Acad Sci, Vol. 108(42) : 17533-17537). A least, CMPF (3-carboxy-4-methyl-5-propyl-2-furanpropanoic acid) a degradation product of FuFAs but also a metabolite derived from the catabolism of PUFAs of the ω-3 family, may contribute to these health effects. CMPF supplementation prevents hepatic steatosis (Prentice et al., 2018, EBioMedicine, Vol. 27 : 200-213).

Furthermore, several studies suggest that FuFAs present in fish may be involved in the beneficial effect of fish consumption on cardiovascular disease (CVD) (Spitteler et al., 2005, Mol Nutr Food Res, Vol. 49(11) : 999-1013). Several studies conducted in humans show that the consumption of fish oil increases the levels of FuFA (Wahl et al., 1994, Hrc-J High Res Chrom, Vol. 17(5) : 308-311) and CMPF in the plasma (Zheng et al., 2016, Sci Rep-Uk, Vol. 6, doi:Ertn 29522). Other studies suggest that, after consumption of fish, FuFAs, incorporated into blood phospholipids and in intimate contact with endothelial cells where LDL oxidation occurs (Puchta et al., 1988, Liebigs Ann Chem, (1) : 25-28), would protect against the development of atherosclerosis.

According to the applicant's knowledge, nothing was known about the biological effects of FuFAs on muscle.

In preferred embodiments, a furan fatty acid of this disclosure is a furan fatty acid of formula (I) described above which is selected from the furan fatty acids wherein,
- n is 2, m is 8, R1 means a methyl group and R2 means a hydrogen atom and R3 means a methyl group, which FuFA is also termed "FuFA-F1" herein, or
- n is 4, m is 8, R1 means a hydrogen atom and R2 means a hydrogen atom and R3 means a methyl group, which FuFA is also termed "FuFA-F2" herein, or
- n is 4, m is 8, R1 means a methyl group and R2 means a hydrogen atom and R3 means a methyl group, which FuFA is also termed "FuFA-F3" herein, or
- n is 2, m is 10, R1 means a methyl group and R2 means a hydrogen atom and R3 means a methyl group, which FuFA is also termed "FuFA-F4" herein, or
- n is 4, m is 10, R1 means a hydrogen atom and R2 means a hydrogen atom and R3 means a methyl group, which FuFA is also termed "FuFA-F5" herein, or
- n is 4, m is 10, R1 means a methyl group and R2 means a hydrogen atom and R3 means a methyl group, which FuFA is also termed "FuFA-F6" herein, or
- n is 4, m is 12, R1 means a hydrogen atom and R2 means a hydrogen atom and R3 means a methyl group, which FuFA is also termed "FuFA-F7" herein, or
- n is 4, m is 12, R1 means a methyl group, and R2 means a hydrogen atom and R3 means a methyl group, which FuFA is also termed "FuFA-F8" herein.

In some preferred embodiments, a furan fatty acid of this disclosure is a furan fatty acid of formula (I) described above wherein, n is 4, m is 8, R1 means a hydrogen atom, R2 means a hydrogen atom and R3 means a methyl group, which FuFA is also termed "FuFA-F2" herein.

In some preferred embodiments, a furan fatty acid-containing compound of this disclosure is of formula (II) below 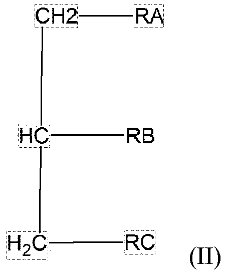 wherein groups RA, RB and RC represent, independently of each other, (i) a hydroxyl group, (ii) -O-C(=O)-R4 , wherein R4 means a group of formula (III) as defined in claim 2 or (iii) -O-C(=O)-R4 , wherein R4 means a (C6-C22) linear or branched, alkyl chain, a (C6-C22) linear or branched alkenyl group or a (C6-C22) linear or branched alkynyl group, provided that at least one of RA, RB and RC represents -O-C(=O)-R4 , wherein R4 means a group of formula (III) as defined in claim 2.

In some embodiments of a furan fatty acid-containing compound of formula (II), RA, RB and RC are identical and each means a furan fatty acid of formula (I) wherein n is 4, m is 8, R1 means a hydrogen atom and R3 means a methyl group.

In some embodiments, of the present disclosure, the subject is a human subject.

In some other embodiments of the present disclosure, the subject is an animal subject, *i.e.* a non-human animal subject, which encompasses vertebrates such as non-human mammals, birds, reptiles, amphibians and fish.

Non-limiting examples of animal subjects include fish, birds, livestock, farm animals, and pets. Animal subjects include backyard animals (such as chickens, turkeys, ducks, geese, pigeons, quail, pheasants, ostriches, emus, rheas and guinea fowl), grazing animals, such as equines (e.g., horses, donkeys, mules, burros), pigs, cattle (e.g., cows, buffaloes, zebus, bison, aurochs, yaks), sheep, goats, camels (e.g., camels, dromedaries, llamas, alpacas), and deer (e.g., reindeer, deer). Pets include companion animals such as felines and canines.

### Provision of furan fatty acids and of furan fatty acid-containing compounds

A plurality of methods for obtaining FuFAs are known in the art.

For preparing FuFAs, the skilled artisan can refer to the published patent n° US 10,399,953 or to the published patent n° US 8,426,620 which both disclose methods of chemical synthesis of FuFAs.

FuFAs may also be prepared as described for example by each of Spiteller (2005, Lipids, Vol. 40(8) : 755-771), by Lemke et al. (2014, Proc Natl Acad Sci USA, Vol. 111(33) : E3450-E3457), Lemke et al. (2020, J Biol Chem, Vol. 295(29) : 9786-9801) Rahn et al. (1979, J Org Chem, Vol. 44(19) : 3420-3424) and Wang et al. (2020, J Org Chem, Vol. 19 : 2914-2922)

FuFAs can also be obtained from a variety of FuFAs-containing natural sources such as fish oil, animal milk-derived fat and plant oil (*e.g*. soybean oil). FuFAs from natural sources can also be obtained from the latex of *Hevea brasiliensis,* such as from the latex of *Hevea brasiliensis.*

Illustratively, FuFAs can be obtained from natural sources by performing solvent extraction methods that are well known in the art, followed by a step of purification by chromatography and, if necessary, a step of saponification so as to obtain FuFAs from trifuranoylglycerol compounds.

In some embodiments, FUFAs can be obtained through a method comprising the steps of :
a) providing a natural substance of interest,
b) extracting the lipids by solvent extraction, whereby an extraction composition is provided,
c) collecting the extracted lipid fraction from the extraction composition obtained at step b), whereby a lipid fraction is provided,
d) removing the solvent from the lipid fraction obtained at step c), whereby a total lipid extract is provided,
e) separating FuFAs, or FuFAs-containing compounds, from the total lipid fraction, whereby a fraction containing separated FuFAs, or FuFAs-containing compounds, is provided,
f) optionally, when the fraction obtained at step e) comprises FuFAs under the form of trifuranoylglycerol compounds, performing a saponification step, whereby a FuFA-containing composition is provided.

At step a) of the method, the natural source of interest can be a latex from *Hevea brasiliensis,* such as from PB 235 or PB 217 clones (Commère et al., 1991, Revue Générale du caoutchouc, Vol. 68(709) : 100-104).

At step b) of the method, solvent extraction can be performed by adding the natural substance of interest into a selected solvent solution, such as adding dropwise freshly harvested latex into a selected solvent solution, such as in a latex: solvent ratio of about 1:5 (v/v), preferably under stirring conditions. The selected solvent can be ethyl acetate. A lipid fraction contained in the extraction mixture is obtained at the end of step b).

At step c), the lipid fraction, which can be located in the upper part of the extraction mixture obtained at step b), is separated, *e.g*. by simply collecting the lipid-containing upper part of the said mixture.

At step d), the solvent which remains in the lipid fraction obtained at step c) is removed, preferably by evaporation, whereby a total lipid extract is provided.

Then, at step e), FuFAs, or FuFAs-containing compounds are separated from the other lipids comprised in the total lipid fraction obtained at step d), preferably through a chromatography separation step. Illustratively, FuFAs, or FuFas-containing compounds can be separated from the other lipids by using a silica gel chromatography column and collecting the FuFAs fraction, or FuFAs-containing compounds fraction.

In some embodiments of the method, the chromatography fraction of interest can comprise FuFAs under the form of FuFAs-containing compounds, especially under the form of FuFAs-containing glycerides, and even more especially under the form of FuFAs-containing triacylglycerols, which may also be termed "trifuranoylglycerols" or "TGFs" in the present disclosure.

FuFAs-containing triacylglycerols are compounds of formula (II) as described herein.

For obtaining FuFAs from trifuranoylglycerols, it is performed the optional step f) of the method comprising saponifying the said trifuranoylglycerols so as to obtain FuFAs.

Such a purification method is illustrated in the examples herein by using a latex from *Hevea brasiliensis* as the natural source of interest. As shown in the examples herein, the FuFAs obtained at step d) of the method, or alternatively at step e) of the method, has more than 97% purity in FuFAs.

In the embodiments wherein the FuFAs or the FuFAs-containing compounds of interest are obtained from natural sources, production of these muscle mass-increasing compounds simply consists of taking benefit from available co-products, notably co-products available from the already existing plant exploitation, such as available from the already existing exploitation from *Hevea brasiliensis,* which is compliant with environment-friendly sustainable development.

In some embodiments, the FuFAs, or the FuFAs-containing compounds, for example those obtained by the purification method above, can be dissolved in an appropriate solvent before being used according to the present disclosure. Illustratively, the FuFAs, or the FuFAs-containing compounds can be dissolved in an appropriate solvent, such as DMSO, as it is shown in the examples herein.

### Compositions comprising FuFAs or FuFAs-containing compounds

For their various uses disclosed herein, FuFAs or FuFAs-containing compounds can be comprised in compositions.

As will be apparent from the description, a composition comprising FuFAs or FuFAs-containing compounds is notably intended for undernourished subjects.

By "undernourished subject", it is meant according to the disclosure a subject presenting a state of undernutrition. The state of undernutrition is well known to the skilled person. The characteristics of a state of undernutrition and its consequences are documented in the introduction to the present disclosure (see also "Diagnosis of undernutrition in children and adults - Method Recommendations for clinical practice", November 2019, Haute Autorité de Santé and Fédération Française de Nutrition, pages 11-12).

Undernourished subjects include humans and animal subjects, including non-human mammals, birds and fish. Undernourished animal subjects include birds, fish, livestock, farm animals, and pets. Undernourished includes backyard animals (such as chickens, turkeys, ducks, geese, pigeons, quail, pheasants, ostriches, emus, rheas and guinea fowl). Undernourished individuals also include grazing animals, such as equines (e.g., horses, donkeys, mules, burros), pigs, cattle (e.g., cows, buffaloes, zebus, bison, aurochs, yaks), sheep, goats, camels (e.g., camels, dromedaries, llamas, alpacas), and deer (e.g., reindeer, deer). Undernourished individuals also include companion animals such as felines and canines.

Undernourished subjects include humans, encompassing children and the elderly who are undernourished.

The present disclosure relates to a nutritional composition comprising a furan fatty acid as described herein, or a furan fatty acid-containing compound as described herein, which is intended for malnourished subjects affected by a loss of muscle mass, said subjects not having a pathology towards which said nutritional composition has a prophylactic and/or therapeutic effect.

As already described elsewhere in the preset disclosure, a furan fatty acid, a furan fatty acid-containing compound, as well as a composition comprising a furan fatty acid or a furan fatty acid-containing compound, can be sued in healthy human or animal subjects for increasing their muscle mass.

For example, a sportsman or sportswoman in whom it is necessary to maintain or increase muscle mass, does not have a pathology and only requires a nutritional contribution. According to another example, a composition as disclosed herein can be a nutritional composition intended for elderly people who are malnourished, or also for overweight people who are malnourished for example due to the practice of a diet.

Another illustration concern animals, which include non-human mammals, birds and fish, for which it may be desirable to increase muscle mass. This other illustration notably relates to farm non-human mammals, birds and fish who are raised for their meat.

A still further illustration relates to human subjects that temporarily live in an environment which may cause a loss in the muscle mass, such as human subjects which evolve in a low gravity environment, sometimes for a six-month time period or even a one-year time period.

By "elderly subject", in the sense of the present disclosure, is meant a human subject or a non-human mammalian subject, including a pet animal such as a dog or cat, which shows signs of senescence such as an alteration of metabolic functions (e.g. absorption, digestion, excretion), locomotor difficulties and reduced resistance to external aggressions.

"Elderly subject" or "elderly person" in the case of a human means a subject who is 65 years of age or older.

"Elderly subject", in relation to a canine, in particular a dog, means a subject who is (i) more than 12 years of age for a canine, in particular a dog, of small size, or (ii) more than 9 years of age for a canine, in particular a dog, of medium size, or (iii) more than 7 years of age, for a canine, in particular a dog, of large size.

By "elderly subject", in the case of a feline, in particular a cat, is meant a subject older than 13 years.

The present disclosure also relates to a pharmaceutical composition comprising a furan fatty acid as described herein, or a furan fatty acid-containing compound as described herein, for its use in subjects having, or likely to have, a pathology on which said composition has a prophylactic or therapeutic effect. By way of example, a composition according to the present description consists of a pharmaceutical composition for its use to prevent or treat a disease state, such as a cachexia linked to a cancer.

The present disclosure further concerns providing a human subject or an animal subject with a furan fatty acid, or a furan fatty acid-containing compound, or composition comprising furan fatty acid, or a furan fatty acid-containing compound, to a subject in need thereof, wherein the human or animal subject is selected among elderly subjects affected by sarcopenia, overweight and obese subjects affected by sarcopenic obesity and subjected to a food regime, subjects affected by cachexia, notably a cachexia linked to cancer, subjects affected by an inflammatory bowel disease or a chronic obstructive pulmonary disease, immobilized subjects, subjects with severe burns and affected with septic or viral infections such as with a SARS-CoV-2 virus, subjects having a heart failure or a respiratory failure, subjects having cirrhosis, subjects affected with a rheumatoid arthritis, convalescent subjects, immobilized subjects, and subjects with bowel resections or intestinal malabsorptions.

However, unless specifically stated, a composition comprising a furan fatty acid as described herein, or a furan fatty acid-containing compound as described herein, does not differ in its general characteristics as set forth in the description as to whether it is a nutritional composition or a therapeutic composition. Thus, in essence, a pharmaceutical composition differs from a nutritional composition in that the pharmaceutical composition has, in the subject to which it is administered, an effect of preventing a disease or an effect of treating a disease.

In some embodiments, a composition for its use according to the present disclosure further comprises one or more vitamins. The vitamins may be vitamin C and vitamins B which encompass vitamin B1 (thiamine), vitamin B2 (riboflavin), vitamin B3 (vitamin PP or niacin), vitamin B5 (pantothenic acid), vitamin B6 (pyridoxine), vitamin B8 (biotin), vitamin B9 (folic acid) and vitamin B12 (cobalamins), and . In some embodiments, the composition comprises one or more vitamins that are fat-soluble, for example one or more of vitamin A, vitamin D, vitamin E, and vitamin K.

In some embodiments, the composition for its use according to the present disclosure comprises one or more polyphenols, such as flavanols, flavanones, flavonols, hydroxycinnamic acids and anthocyanins.

In some embodiments, the composition for its use according to the present disclosure further comprises one or more minerals. The minerals may be selected from sodium, potassium, chloride, calcium, phosphate, magnesium, iron, zinc, copper, selenium, manganese, fluorine, iodine, chromium or molybdenum. Minerals are usually added in the form of salt. The minerals may be added alone or in combination.

In some embodiments, a composition for its use according to the present disclosure can include carriers or vehicles. "Carriers" or "vehicles" refer to materials suitable for administration and include any material known in the art, such as, for example, any liquid, gel, solvent, liquid diluent, solubilizing agent, or the like, that is non-toxic and does not interact with the components of the composition in a deleterious manner. Examples of nutritionally acceptable carriers include, for example, water, saline solutions, alcohols, silicones, waxes, petrolatum, vegetable oils, polyethylene glycols, propylene glycol, liposomes, sugars, gelatin, lactose, amylose, magnesium stearate, talc, surfactants, silicic acid, viscous kerosene, perfume oil, monoglycerides and diglycerides of fatty acids, petroleum fatty acid esters, hydroxymethyl cellulose, polyvinyl pyrrolidone, etc..

In some embodiments, the composition for its use according to the present disclosure further comprises any other ingredient or excipient known to be employed in the type of composition in question. Non-limiting examples of such ingredients include: proteins, amino acids, carbohydrates, oligosaccharides, lipids, nucleotides, nucleosides, other vitamins, minerals and other micronutrients.

In some embodiments, the composition for its use according to the present disclosure contains a source of carbohydrate, for example in the form of prebiotics, or if prebiotics are present in the composition, they are present. Any carbohydrate source typically found in infant formula, such as lactose, sucrose, maltodextrin, starch, and mixtures thereof, may be used, although the preferred carbohydrate source is lactose.

In some embodiments, a composition for its use according to the present disclosure consists of a nutritional composition.

In some embodiments, such a nutritional composition is selected from complete food compositions, dietary supplements, nutraceutical compositions and the like. The composition of the present description may be used as a food ingredient. The food ingredient may be in the form of a solution or a solid, depending on the use and/or method of application and/or method of administration. As used herein, the term "food" refers to liquid (i.e., beverages), solid, or semi-solid dietary compositions, particularly total food (food replacement) compositions, which do not require additional nutrient intake or dietary supplement compositions. Food supplement compositions do not completely replace the supply of nutrients by other means. As used in this disclosure, the term "dietary ingredient" includes a formulation that is or may be added to functional foods or foodstuffs as a dietary supplement. The term "nutritional food" or "nutraceutical" or "functional" means a food material that contains ingredients that have health benefits or are capable of improving physiological functions. "Food supplement" means a food material that is intended to supplement a normal diet. A dietary supplement is a concentrated source of nutrients or other substances having a nutritional or physiological effect, when taken alone or in combination in small amounts. As used herein, "functional food" is used to refer to food materials and corresponding products that are valued not only for their nutritional and taste value but also for the presence of ingredients with beneficial physiological effects.

In some embodiments, the composition for its use according to the present disclosure is a fermented dairy or milk-based product, which is preferably administered or ingested orally one or more times per day. Fermented dairy products include milk-based products, such as (but not limited to) desserts, yogurt, yogurt drinks, cottage cheese, kefir, fermented milk beverages, buttermilk, cheeses, salad dressings, low-fat spreads, cream cheese, soy-based beverages, ice cream, etc.

Thus, in some embodiments, a furan fatty acid, or a furan fatty acid-containing compound, is comprised in an oral composition, and more particularly in an oral composition selected from the group consisting of a food product, a beverage, a pharmaceutical, a nutraceutical, a food additive, a dietary supplement, a dairy product and a living biotherapy product (LBPs).

Alternatively, in some embodiments, the nutritional and/or nutritional supplement compositions for their use according to the present disclosure can be non-dairy or non-fermented dairy products. Non-dairy products may include ice cream, nutrition bars and seasonings, and the like. Non-dairy products may include powdered drinks and nutrition bars, etc. The products can be made using known methods, such as adding an effective amount of a furan fatty acid as described herein, or an effective amount of a furan fatty acid-containing compound as described herein, to a food base, such as skim milk or milk or a milk-based composition, and performing fermentation according to any known technique. In some embodiments, the composition is a beverage which may be a functional or therapeutic beverage, a conventional non-therapeutic beverage. By way of example, the composition for its use according to the present disclosure can be used as an ingredient for carbonated beverages, a fruit juice or a beverage comprising whey protein, teas, cocoa drinks, milk drinks, yogurts including yogurt drinks, cheeses, ice creams, popsicles and desserts, confectionery, cookies, cakes and cake mixes, snacks, balanced foods and drinks, icings, soy drink/acidified juice, aseptic/reduced chocolate drink, bar mixes, powdered drink mixes, calcium-enriched soy milk and chocolate, calcium-enriched coffee drink.

In some embodiments, the composition includes any other ingredient or excipient known to be employed in the type of composition. Non-limiting examples of such ingredients include : proteins, amino acids, carbohydrates, oligosaccharides, lipids, prebiotics or probiotics, nucleotides, nucleosides, other vitamins, minerals and other micronutrients.

In some other embodiments, a furan fatty acid as described herein, or a furan fatty acid-containing compound as described herein, is administered to the subject in the form of a pharmaceutical composition, which could correspond to a Live Biotherapeutic product (LBP) reference: Front Med (Lausanne) Rouanet et al2020 Jun 19;7:237. doi: 10.3389/fmed.2020.00237 . For example, a furan fatty acid of interest as described herein, or a furan fatty acid-containing compound of interest as described herein, can be combined with pharmaceutically acceptable excipients, and optionally sustained release matrices, such as biodegradable polymers, to form therapeutic compositions. The terms "pharmaceutically" or "pharmaceutically acceptable" refer to molecular entities and compositions that do not produce an adverse reaction, allergic reaction, or the like when administered to a mammal, particularly a human, as appropriate. A pharmaceutically acceptable carrier or excipient means a non-toxic solid, semi-solid or liquid filler, diluent, encapsulating material or formulation aid of any type. In the pharmaceutical compositions of the present description for oral, sublingual, subcutaneous, intramuscular, intravenous, transdermal, local or rectal administration, the active ingredient or combination of active ingredients may be administered in a unitary administration form, in admixture with conventional pharmaceutical carriers, to animals and humans. Suitable unit forms of administration include oral forms of administration such as tablets, gel capsules, powders, granules and oral suspensions or solutions, sublingual and buccal forms of administration, aerosols, implants, subcutaneous, transdermal, topical, intraperitoneal, intramuscular, intravenous, subcutaneous, transdermal, intrathecal and intranasal forms of administration and rectal forms of administration. Typically, the pharmaceutical compositions contain vehicles that are pharmaceutically acceptable for an injectable formulation. These may be, in particular, sterile, isotonic saline solutions (monosodium or disodium phosphate, sodium, potassium, calcium or magnesium chloride and the like, or mixtures of these salts), or dry compositions, in particular freeze-dried compositions, which, by adding sterilized water or physiological saline, as the case may be, allow the formation of injectable solutions. Pharmaceutical forms suitable for injectable use include sterile aqueous solutions or dispersions; formulations comprising sesame oil, peanut oil or aqueous propylene glycol; and sterile powders for the extemporaneous preparation of injectable sterile solutions or dispersions. In all cases, the form must be sterile and must be fluid to the point of being easily squeezed.

The pharmaceutical composition for its use according to the present disclosure must preferably be stable under manufacturing and storage conditions and must preferably be preserved from the contaminating action of microorganisms, such as bacteria and fungi. Solutions comprising a furan fatty acid or a furan fatty acid-containing compound as disclosed herein, in free base or pharmacologically acceptable salt form, may be prepared in water suitably mixed with a surfactant, such as hydroxypropyl cellulose. Dispersions may also be prepared in glycerol, liquid polyethylene glycols and mixtures thereof, and oils. Under ordinary conditions of storage and use, these preparations contain a preservative to prevent the growth of microorganisms. A furan fatty acid as described herein, or a furan fatty acid-containing compound as described herein, may be formulated in a composition in a neutral form or as a salt. Pharmaceutically acceptable salts include acid addition salts (formed with the free amino groups of the protein) and which are formed with inorganic acids such as, for example, hydrochloric or phosphoric acids, or organic acids such as acetic, oxalic, tartaric, mandelic, and the like. The salts formed with the free carboxyl groups can also be derived from inorganic bases such as, for example, sodium, potassium, ammonium, calcium, or ferric hydroxides, and from organic bases such as isopropylamine, trimethylamine, histidine, procaine, and the like.

As used herein, the term "therapeutically effective amount" is an equivalent term that refers to the amount of a therapy (e.g., a prophylactic or therapeutic agent) that is sufficient to reduce the severity and/or duration of a disease state, ameliorate one or more of its symptoms, prevent the progression of a disease, or cause the regression of a disease or which is sufficient to result in the prevention of the development, recurrence, onset or progression of a disease or one or more of its symptoms, or to enhance or improve the prophylactic and/or therapeutic effect(s) of another therapy (e.g., another therapeutic agent) useful for treating a disease. Typically, in a pharmaceutical composition for its use according to the present disclosure, which comprises a furan fatty acid as described herein, or a furan fatty acid-containing compound as described herein, is present in an amount sufficient to prevent a loss in muscle mass, or to treat a loss of muscle mass in a mammal subject, preferably in a human, such as to prevent or to treat sarcopenia in the said mammal subject, preferably in the said human subject.

The amount of a furan fatty acid as described herein, or a furan fatty acid-containing compound as described herein, that is provided to the subject may be variable, depending on the physiological state of said subject, and in particular depending on the level of imbalance of nutrient intake versus nutrient requirement of said subject. The amount of a furan fatty acid as described herein, or of a furan fatty acid-containing compound as described herein, to be supplied to said subject can be easily adapted by the person skilled in the art.

Illustratively, a composition for its use according to the present disclosure, either being a nutritional composition or a pharmaceutical composition, can be administered to the mammal subject in need thereof, preferably to the human subject in need thereof, preferably by the oral route, at a daily amount of a furan fatty acid ranging from about 100 ng per kilogram of body weight to about 1 g per kilogram of body weight, such as ranging from about 1 mg per kilogram of body weight to about 100 mg per kilogram of body weight, such as for example at about 20 mg per kilogram of body weight.

The amount of active compounds to be administered is based on the weight of furan fatty acids *per se.* For example, the amount of a furan fatty acid-containing compound to be administered is determined as regards the amount of furan fatty acid comprised therein.

Such a pharmaceutical composition may be presented in the form of a package comprising a plurality of dosage units.

The term "dosage unit" is used in its conventional pharmaceutical sense (e.g., a pill, a capsule, a tablet, the contents of an ampoule, etc.).

### Uses of furan fatty acids and of furan fatty acid-containing compounds

The present disclosure relates to nutritional uses and therapeutic uses of furan fatty acids and furan fatty acid-containing compounds as defined in the present application.

The characteristics of compositions, including a nutritional composition and a therapeutic composition, including in various embodiments, are described in detail in the present description, which includes the amounts of active ingredient(s), especially the amounts of a furan fatty acid as described herein, or a furan fatty acid-containing compound as described herein, included in these compositions.

The present description relates to the use of a composition as defined in the present application to maintain or increase muscle mass and/or muscle function in a subject in need thereof.

The present description further pertains to the non-therapeutic use of a furan fatty acid, or of a furan fatty acid-containing compound, for maintaining or increasing muscle mass in a subject in need thereof, in particular in a subject selected from a malnourished subject, an elderly subject, in particular a malnourished elderly subject, and a subject engaged in strenuous exercise.

In some embodiments of the said non-therapeutic use, the furan fatty acid, or the furan fatty acid-containing compound is of formula (I) or (II) as according to the present disclosure.

In many embodiments, a composition for its use according to the present disclosure consists of a nutritional composition, *i.e.,* a composition that is used for nutritional purposes to maintain or increase muscle mass in a subject in need thereof. Such a use remains a nutritional use of a non-therapeutic nature, including in certain sick subjects whose pathology they are affected by is concomitant with a loss of muscle mass. Indeed, in these contexts, the contribution of a nutritional composition according to the present description does not have the effect of preventing or treating the pathology of which the subject is affected, but rather has the effect of providing a nutritional benefit to the said subject who is otherwise receiving a therapeutic treatment intended to cure the said pathology.

However, the regulatory provisions may vary according to the countries concerned, which means that a composition according to the present description, in its modes of use with nutritional effects described above, may, if necessary, be considered from a regulatory point of view governing health law, as a medicine.

The present description relates to the use of a nutritional composition as defined in the present application to maintain or increase muscle mass in a subject in need thereof. Preferably, subjects in need of a nutritional composition as defined in the present application are selected from undernourished subjects, elderly subjects, particularly elderly denuded subjects, subjects engaged in strenuous exercise or subjects with increased protein requirements, subjects that undergo physical immobilization, for example due to a stay at an hospital or at home, or in an astronautical craft.

In some embodiments, said nutritional composition consists of a nutritional supplement composition as defined in the present disclosure.

In some embodiments, a nutritional composition for its use according to the present disclosure, optionally in the form of a dietary supplement composition, is formulated for daily administration, for example for daily administration in one or two doses, for example at mealtimes.

In general, a nutritional composition for its use according to the present disclosure may be administered to the subject until the value of muscle mass and/or muscle function reaches a predetermined level, indicative of normal muscle mass or even until the value of muscle mass and/or muscle function exceeds a normal muscle mass (see for example "Diagnosis of undernutrition in children and adults - Method Recommendations for clinical practice", November 2019, Haute Autorité de Santé and Fédération Française de Nutrition, the table at the end of page 11). As an illustration, the level of muscle mass and/or muscle function can be determined with regard to at least one of the following criteria: (i) grip strength measured by a dynamometer, for example in kg, (ii) walking speed, for example measured in m/s, (iii) the muscle area index in L3, for example measured in cm²/m², for example using a scanner device such as MRI, (iv) muscle mass index, e.g. in kg/m², e.g. measured by impedancemetry, (v) nonfat mass index, e.g. in kg/m², e.g. measured by impedancemetry, or (vi) appendicular muscle mass (DEXA), e.g. measured in kg/m². The measurement of muscle metabolic dynamics (which results in the regulation of muscle mass and function) can also, in some cases, be assessed using more invasive techniques, for example, techniques to measure the rate of protein turnover by injection of amino acids marked by stable isotopes.

In some embodiments, a nutritional composition according to the present description is administered to the subject chronically, over a long period of time, for example, over several months or even several years, for the purpose of maintaining the level of muscle mass and/or muscle function at a predetermined value indicative of the absence of normal muscle mass and/or normal muscle function.

The present description also relates to medical uses of a composition as described in the present application.

The description relates to a composition as defined in the present application for its use as a medicament.

It relates to the use of a composition as defined in the present application for the manufacture of a medicament.

The description also relates to a composition as defined in the present application for use in preventing or treating loss of muscle mass in subjects in need thereof. Preferably, the subjects in need are chosen from overweight and obese subjects, and subjected to a hypocaloric and/or hypoglucidic diet, obese subjects or not having undergone bariatric surgery, who therefore have a sharp decrease in their overall energy intake but whose protein intake must be maintained to avoid a loss of muscle mass, cancer patients, in particular cancer patients with cachexia, patients treated with antibiotics, patients with viral or bacterial infections, diabetic patients on a low-calorie and/or low-carbohydrate diet, or patients in intensive care who are fed enteral feeding.

The description also relates to the use of a composition as defined herein for the manufacture of a medicament for preventing or treating loss of muscle mass in subjects in need thereof. Preferably, the subjects in need are selected from overweight subjects and obese subjects subjected to a hypocaloric diet, obese or non-obese subjects having undergone bariatric surgery cancerous subjects, in particular cancerous subjects affected by sarcopenia, subjects treated with antibiotics, subjects affected by a viral or bacterial infection, subjects affected by sarcopenia, diabetic subjects subjected to a hypocaloric diet, or in subjects in intensive care who are fed by enteral route.

The description also relates to a method for preventing or treating loss of muscle mass and function in subjects in need thereof comprising administering to said subject a composition as defined herein.

Thus, in some embodiments, a composition as defined in the present application may be used as a preventive composition. For example, the composition may be administered, as a preventive measure, to subjects at risk of developing loss of muscle mass and function, which includes obese subjects undergoing weight loss treatment, for whom loss of muscle mass and/or loss of muscle function has not occurred.

Generally, a therapeutic composition according to the present description may be administered to the subject until the value of muscle mass and/or muscle function reaches a predetermined level, indicative of an absence of sarcopenia

In some embodiments, a therapeutic composition according to the present description is administered to the subject chronically, over a long period of time, such as over several months or even several years, for the purpose of maintaining the value of muscle mass and/or muscle function at a predetermined level, indicative of the absence of sarcopenia.

The present disclosure is further illustrated by, without in any way being limited to, the examples below.

### EXAMPLES

### A. MATERIALS AND METHODS

### A.1 FuFA-F2 purification

Fresh latex from PB235 *Hevea brasiliensis* clone was collected from a plantation of Visahakit Thai Rubber Co., Ltd., Chanthaburi, Thailand. Lipid extraction was performed by adding freshly harvested latex dropwise into continuously stirred ethyl acetate (Univar, Illinois, USA) with a ratio latex: solvent of 1:5 (v/v). Coagulum made mainly of insoluble polyisoprene was removed by filtration and the extract was let decanted in separating funnel overnight. The upper fraction of lipid-containing ethyl acetate was collected and evaporated. Total lipid extract was further purified using silica gel column chromatography. Lipid extract (1.8 g of lipid solution in hexane) was loaded onto a silica gel column (column 4.0 cm diameter x 16 cm length) containing 42 g of silica gel 60 (0.040-0.063 mm, Merck, Germany). Mobile phase was made of 760 mL of hexane:diethyl ether (90:1, v/v) at the flow rate of 1.8 mL/min and the collection volume was 19 mL/fraction. The separation quality was checked by spotting an aliquot of each fraction on a thin layer chromatography plate following the method described for neutral lipid by Liengprayoon et al (2013, Eur J Lipid Sci Tech, Vol. 115(9) : 1021-1031). The selected fractions containing only Trifuranoylglycerols (TGF) were saponified and the purity of the produced free FuFA-F2 was analyzed by GC-FID on methyl ester derivatives as described previously (Liengprayoon et al, 2013, Eur J Lipid Sci Tech, Vol. 115(9) : 1021-1031). The purity of FuFA-F2 used in this study was more than 97%. FuFA-F2 under the free acid form was dissolved in DMSO before used in C2C12.

### A. 2 Animals and Ethics statement

Male C57Bl/6J wild-type mice were obtained from Janvier at 12 weeks of age and were randomly assigned to one of the two groups based on body weight. Mice were housed 2 per cage and maintained on a 12-hour light/dark cycle (lights on at 7:30 pm). Food and water were provided ad libitum. Enrichment (nesting cotton squares) was provided in each cage. Mice were treated once daily by oral gavage with vehicle (50% PEG-400, 0.5% Tween 80, and 49.5% distilled water) or FuFA-F2 (20 mg/kg). All animal experiments were performed according to European directives (86/609/CEE and 2010/63/CEE) and approved by the regional animal experimentation ethics committe: Région Languedoc-Roussillon (No36). Our institutional guidelines for the care and use of laboratory animals were observed. Our animal facility is approved by the Departmental Veterinary Services (No. E34-172-10, 2019/03/04) and the Ministry of Research (DUO No. 7053, 2020/02/26). according to the European Directive 210-63-EU, mice were observed daily for the general health status and mortality scoring.

### A.3 Protein synthesis measurement

Protein synthesis was measured by determining puromycin incorporation into proteins as previously described (Goodman et al., 2013, Exercise and Sport Sciences Reviews, Vol. 41(2) : 107-115). In this aim, mice were intraperitoneally injected with 10 mM puromycin (100 µL of puromycin solution/25 g body weight, Sigma Aldrich, Saint-Louis, Missouri, USA). Twenty-five minutes after puromycin injection, animals were euthanized. Puromycin incorporation in proteins was assessed by immunoblotting on 4-20% acrylamide gels and using an anti-puromycin primary antibody (anti-puromycin antibody (1/3000), clone 12D10 from EMD Millipore, Burlington, Massassuchets, USA). The optical density of the entire sample lane was assessed and normalized with Ponceau S total protein staining (Roumanille et al., 2020, J Int Sport Nutr, Vol. 17(1) : doi:Artn 58).

### A.4 Cell Culture and Treatments

Mouse myoblasts of the C2C12 cell line (ATCC) were seeded at a plating density of 7000 cells/cm2 in 6-well plates. They were grown in DMEM high glucose (4.5g/l) supplemented with gentamycin (50 µg/mL), amphotericin (50 µg/mL), and fetal calf serum (10%). Terminal differentiation was induced at cell confluence by lowering the medium serum concentration (0.5%).

When indicated, FuFA-F2 was added to the culture medium at the indicated concentration. For the differentiation studies, FuFA-F2 was added at the confluence at the same time as the change in media condition. Three doses of FuFA-F2 were studied at a final concentration of 1, 10, or 50 µM. Human insulin was used at a concentration of 100 nM.

### A.5 Cytoimmunofluorescence

Cytoimmunofluorescence myoblast differentiation was assessed by observation of morphological changes and accumulation of muscle-specific markers. After methanol fixation and three washes with PBS-gelatin (0.2%), cells were labeled with an antibody raised against Troponin T (T6277, Sigma, diluted at 1:50) (Sigma-Aldrich, Saint-Quentin Fallavier, France). Nuclei were stained with Hoechst 33258 (1 µg/mL). To assess the extent of differentiation, the fusion index (percentage of nuclei incorporated into myotubes relative to the total number of nuclei) was calculated 120 h after adding the differentiation medium. Nuclei were counted on 10 images/dish using ImageJ software.

### A.6 Western blotting

Protein levels were assessed by Western blotting. Total proteins were measured using the Bio-Rad protein assay (Hercules, CA, USA) according to the manufacturer's instructions. Total proteins were lysed in RIPA buffer (20mM Tris, 150mM NaCl, 1mM EDTA, 1% NP40, pH 7.5 + complete cocktail protease inhibitors + Pierce phosphatase inhibitors tablet). 40 µg of cell extracts were loaded and blotted onto a nitrocellulose membrane. The membranes were washed, blocked with 5% nonfat milk and incubated in primary antibody overnight at 4°C. The membranes were then washed in TBS-Tween (10mM Tris, 140mM NaCl, and 0.2% Tween-20), incubated with the appropriate secondary antibody coupled to horseradish peroxidase, and washed again in TBS-Tween. Signals were revealed using a Clarity^{™} Western ECL Substrate Kit (Hercules, CA, USA), and proteins were visualized by enhanced chemiluminescence using the ChemiDoc Touch Imaging System (Hercules, CA, USA) and quantified with Image Lab. Touch Software (version 5.2.1) (Hercules, CA, USA).

### A.7 Antibodies

Primary antibodies used include: anti-AMPK (1/500, Cell Signaling #2603) (Cell Signaling Technology, Leiden, Netherlands); anti-Phospho-AMPK (Thr172) (1/500, Cell Signaling #2535); anti-Akt (1/500, Cell Signaling #9272); anti-Phospho-Akt (Ser473) (1/500, Cell Signaling #9271); anti-ULK1 (1/500, Cell Signaling #8054); anti-Phospho-ULK1 (Ser757) (1/500, Cell Signaling #6888); anti-mTOR (1/500, Cell Signaling #2972); anti-Phospho-mTOR (Ser2448) (1/500, Cell Signaling #2971); anti-myogenin (1/500, Santa Cruz Biotechnology Sc-398002) (Santa Cruz Biotechnology, Heidelberg, Germany); anti-LC3A (1/1000, Cell Signaling #4599); anti-4E-BP1 (1/500, Cell Signaling #9644); anti-Phospho-4E-BP1 (Thr37/46) (1/500, Cell Signaling #2855); Anti-α-Tubulin (1/6000, Cell Signaling #3873); Anti-Myosin (Fast) (1/1000, Sigma M4276); Anti-Myosin (Slow) (1/1000, Sigma M8421) (Sigma-Aldrich, Saint-Quentin Fallavier, France).

### A.8 Gene Expression Studies

Total RNAs were extracted from quadriceps muscle using Trizol^{®}, and cDNAs were generated using the PrimeScript- 1^{st} strand cDNA Synthesis Kit (Takara Bio, Saint-Germain-en-Laye, France). Real-time PCR was performed using SYBR^{®} Premix Ex Taq- II (Takara Bio) and Applied Biosystems Step-One Plus (Thermo Fisher Scientific, Waltham, MA, USA). Gene expression was normalized to the expression of the housekeeping gene Rps9. The primers for the determination of MyHC were previously described (Casas et al., 2008, PloS ONE, Vol. 3(6) : e2501).

The PCR overall efficiency (E) was calculated from the slopes of the standard curves according to the equation E = [10^{(-1/slope)}] - 1, and this value was higher than 95% for all assays. The relative abundance of each sample was then normalized according to the equation: relative quantity = 2^{- □Ct}. All of the experiments were performed according to the minimum information for publication of quantitative real-time PCR experiment (MIQE) guidelines (Bustin et al., 2009, Clin Chem, Vol. 55(4) : 611-622; Schmittgen et al., 2008, Net Protoc, Vol. 3(6) : 1101-1108).

### A.9 Mitochondrial Enzymatic Activities

Mitochondrial respiratory complex activities were determined as previously described (Casas et al., 2008, PloS ONE, Vol. 3(6) : e2501). Citrate synthase (CS) activity was measured according to Srere (Srere et al., 1967, Science, Vol. 158(3803) : 936-937) by following the color formation of 5-thio-2-nitrobenzoic acid at 412 nm. The enzymatic activity of complex IV (cytochrome c oxidase) was measured according to Wharton and Tzagoloff (Wharton et al., 1967, Methods in Enzymology, Vol. 10, Edn Elsevier BV, Amsterdam, The Netherlands), a spectrophotometer following the oxidation of reduced cytochrome c at 550 nm, pH 7.4, and 37 °C. Cytochrome c was reduced with sodium dithionite.

### A.10 Statistical analyses

All results are presented as means ± SEM, or as percentages. Statistical significances of the differences compared to Controls were evaluated with Student's t-test.

### B. RESULTS

### Example 1 : In vitro effects of FuFAs on muscle metabolism

### 1.1 FuFA-F2 supplementation has little influence on C2C12 myoblast differentiation

For *in vitro* studies, the C2C12 cell line is classically used because it recapitulates the different phases observed *in vivo*: proliferation, withdrawal from the cell cycle, and fusion into myotubes. After a proliferation phase, the C2C12 myoblasts reach confluence and their terminal differentiation is induced by lowering the serum level in the culture medium. C2C12 cells were grown for 5 days in a differentiation medium, with or without FuFA-F2 (1, 10, and 50 µM) (Figure 1A). In humans, the levels of FuFAs in plasma are around 1 µM (Puchta et al., 1988, Liebigs Ann Chem, Vol. 1 : 25-28; Wahl et al., 1994, Hrc-JH High Res Chrom, Vol. 17(5) : 308-311; Xu et al., 2020, Journal of Lipid Research, Vol. 61(4) : 560-569). The results show that FuFA-F2 had no significant effects on myoblast fusion index except for the highest dose of 50 µM, where a slight increase in differentiation was shown (61% with FuFA vs 57% for control, p<0.05) (Figure 1A). Consistent with this observation, FuFA-F2 had no effect on the expression of myogenin, a key myogenic factor for differentiation (Figure 1B).

### 1.2. FuFA-F2 supplementation increases protein accretion in C2C12 myotubes

Next were employed C2C12 myotubes that had been differentiated from myoblasts in order to test the influence of FuFA-F2 at this stage (Figure 2A). It was observed that whatever the dose, 3 days of FuFA-F2 supplementation significantly increase total protein content in C2C12 myotubes (Figure 2B).

### 1.3 : FuFA-F2 supplementation decreases autophagy in C2C12 myotubes

It is shown that FuFA-F2 increased protein accretion in myotubes. Protein homeostasis is determined by the dynamic balance between protein synthesis and degradation. Protein synthesis was evaluated by measuring the phosphorylation of Akt (Ser473), mTOR (Ser2448) and 4E-BP1 (Thr37/46), which are major regulators of translation and protein synthesis. Unfortunately, basal level of Akt and 4E-BP1 phosphorylation was undetectable with C2C12 myotubes in the present experiments (Figure 3A). In addition, it was found no difference in mTOR phosphorylation with or without FuFA-F2 (Figure 3A-B) perhaps because a rapid down regulation of this pathway occurred in high glucose medium.

AMP activated protein kinase (AMPK) is a crucial cellular energy sensor protein and is activated when intracellular ATP level decreases to regulate autophagy initiation (Hardie et al., 2012, Nat Rev Mol Cell Bio, Vol. 13 (4) : 251-262). Activation level of AMPK as assessed by phosphorylation at Thr172 was significantly decreased by FuFA-F2 whatever the dose (Figure 3A-B). The autophagy-related signaling was further evaluated by measuring the MTORC1 mediated phosphorylation by ULK1 at Ser757 which disrupts the interaction between ULK1 and AMPK. Surprisingly ULK1 phosphorylation was decreased for the highest dose of FuFA-F2, however the observation that total Ulk level was also decreased was not in favor of autophagy activation (Figure 3C-D). Therefore, the autophagic flux was assessed by measuring the conversion of LC3-I to LC3-II, through lipidation. It was found that the ratio of LC3-II/LC3-I was decreased by FuFA-F2 whatever the dose (Figure 3C-D) suggesting that FuFA-F2 inhibited the autophagy process in C2C12 myotubes.

### 1.4. FuFA-F2 supplementation increase mTOR phosphorylation

The observation that basal level of Akt phosphorylation was undetectable with C2C12 myotubes in the present experiments cultivated with a classical high glucose medium suggested a rapid down regulation of this pathway masking a potential activation. A similar hypothesis may also be done for mTOR activation. To avoid this possibility, C2C12 myotubes were treated as before and then cultured them for 2 hours in low-glucose medium before adding again the different treatments as indicated for 30 minutes (Figure 4A). In this condition, it was shown that insulin,used as positive control, induced a strong increase in Akt phosphorylation at Ser473 (Figure 4B-C). Compared to low glucose medium, the normal high-glucose medium and FuFA-F2 induced a similar increase of Akt phosphorylation (Figure 4B-C). In addition, treatment with FuFA-F2 did not alter insulin-induced phosphorylation of Akt (Figure 4B-C). This indicates that FuFA-F2 treatment does not appear to regulate Akt phosphorylation. Furthermore, in FuFA-F2 treated myoblasts it was observed a significant increase in mTOR phosphorylation at Ser2448 while no increase was detected in the presence of insulin alone (Figure 4B-C). This data set suggests that FuFA-F2 stimulates mTOR phosphorylation and protein synthesis.

To determine whether mTOR phosphorylation is induced only by 30 min of incubation in the presence of FuFA-F2 or whether it is an effect partly related to the 3-day treatment with FuFA-F2, the experiment was repeated without pretreatment (Figure 4D). In this condition it was still observed that only insulin is able to induce a strong increase in Akt phosphorylation (Figure 4E-F). However, in contrast to the previous experiment a rapid effect of FuFA-F2 on mTOR phosphorylation was not observed (Figure 4E-F). This observation suggests that the activation of mTOR by FuFA-F2 was related to the 3-day treatment.

### 1.5. FuFA-F2 supplementation modulates the metabolic and contractile characteristics of C2C12 myotubes

It was further explored whether this increase in myotubes anabolism is associated with a change in the metabolic and contractile characteristics of muscle fibers. To address this issue, the influence of FuFA-F2 on mitochondrial activity in C2C12 myotubes was tested. It was found that 3 days of FuFA-F2 treatment of C2C12 myotubes had no effect on citrate synthase activity

(Figure 5A), commonly used as a quantitative marker of intact mitochondria. However, at the 10 µM dose, FuFA-F2 tended to increase the Complex IV activity (+60 %, p<0.07) (Figure 5B), a key enzymatic activity of the mitochondrial respiratory chain. To examine whether this trend of increased of mitochondrial respiratory chain activity with 10 µM FuFA-F2 was associated with a change in the contractile type of myosin heavy chain (MyHC), the expression of slow and fast MyHC by western-blot was studied. It was found that at the 10 µM dose, FuFA-F2 significantly increased the expression of slow MyHC which are characteristic of high mitochondrial activity (Figure 5C-D). Surprisingly, a decrease of fast MyHC was observed for the highest dose of FuFA-F2 (Figure 5C-D).

### Example 2 : In vivo effects of FuFAs on muscle metabolism

### 2.1. FuFA-F2 supplementation in C57B16 mice increase muscle mass

To investigate *in vivo* the potential impact a furan-containing compound under the form of a triacylgycerol of FuFA-F2 on muscle characteristics, C57Bl6 mice were supplemented by oral gavage with vehicle or FuFA-F2 (20mg/kg) for 3 and a half weeks (Fig. 6A). It was observed that mice receiving FuFA-F2 ate more and had a significantly higher body weight than animals receiving the vehicle alone (Fig. 6B-C). In addition, analysis of the body composition using an EchoMRI revealed that the mice supplemented with FuFA-F2 had a greater lean mass than the control mice (Fig. 6D). However, fat mass was similar between the two groups (Fig. 6E). In agreement with these observations, the weight of the quadriceps and gastrocnemius, the largest muscles of the hindlimbs, is significantly increased in mice receiving FuFA-F2 (Fig. 6F). These data demonstrated that 3 weeks of FuFA-F2 supplementation was sufficient to stimulate muscle anabolism in mice.

### 2.2. FuFA-F2 supplementation promotes more oxidative muscle metabolism in mice

To determine if this increase in muscle mass is accompanied by changes in the contractile and metabolic characteristics of the muscle fibers, the expression of myosin heavy chains and mitochondrial activity in the quadriceps was measured. The results did not indicate any significant difference in the expression of the different myosin heavy chains between mice treated or not with FuFA. However, a trend towards an increase in MyHCIIa expression at the expense of MyHCIIb with FuFA supplementation was observed (Fig. 7A). Citrate synthase (CS), a marker of mitochondrial activity, was not modified (Fig. 7B). It was found a significant increase of the complex IV activity of mitochondrial respiratory chain complexes in FuFA group (Fig. 7C). These set of data indicates that FuFA-F2 supplementation promotes more oxidative muscle metabolism in mice.

### Summary of the results shown in the examples 1 and 2

Several studies suggest that FuFAs could have beneficial health effects. However, only a few studies have been performed to date.

In the present study, it was tested for the first time the influence of FuFA-F2 extracted from *Hevea brasiliensis* latex (Liengprayoon et al., 2011, Phytochemistry, Vol. 72(14-15) : 1902-1913) on skeletal muscle phenotype. To address this potential effect of FuFA-F2 cultured skeletal muscle cells were used. Mouse C2C12 skeletal muscle cell lines are commonly used to study muscle differentiation, muscle hypertrophy and atrophy. In this work the use of 3 different doses of FuFA-F2 (1, 10 and 50 µM) was chosen. The lowest dose of 1µM is comparable to the concentration found in human plasma (Puchta et al., 1988, Liebigs Ann Chem (1) : 25-28; Wahl et al., 1994, Hrc J High Res Chrom, Vol. 17(5) : 308-311; Xu et al., 2020, Journal of Lipid Research, Vol. 61(4) : 560-569).

First, the influence of FuFA-F2 on the myoblasts differentiation was tested. It was found that FuFA-F2 had no effects on myoblast fusion index except for the highest dose of 50 µM, where a slight increase in differentiation was shown. Then, the effect of FuFA-F2 supplementation on C2C12 myotubes was studied. It was shown that 3 days of FuFA-F2 treatment significantly increase total protein content in C2C12 myotubes whatever the dose. This observation indicates clearly that FuFA-F2 increased protein accretion in myotubes. To understand how FuFA-F2 induces an increase in total protein content and muscle anabolism protein homeostasis was investigated. The dynamic balance between protein synthesis and degradation determines protein homeostasis. It was first explored the synthesis pathways but in a classical hyperglycemic medium it was not possible to detect Akt phosphorylation and no difference on mTOR activation with FuFA-F2 treatment was seen. It was hypothesized that the high glucose medium could mask a possible effect by inducing a rapid down regulation of Akt/mTOR pathway. To avoid this possibility, C2C12 myotubes were cultured for 2 hours in low-glucose medium before adding the different treatments for 30 minutes. Under this condition it was observed that 3 days of FuFA-F2 treatment induced a strong increase in mTOR phosphorylation. This observation indicates that FuFA-F2 stimulates protein synthesis. However, in these experiments any influence of FuFA-F2 on Akt phosphorylation was observed. Although the classical pathway of mTOR regulation involves Akt, other mechanisms of activation of this pathway have already been shown (Memmott et al., 2009, Cellular Signalling, Vol. 21(5) : 656-664). In particular, several studies indicate that the LKB1/AMPK pathway inhibits mTORC1 (Carretero et al., 2007, Oncogene, Vol. 26(11) : 1616-1625; Gwinn et al., 2008, Molecular Cell, Vol. 30(2) : 214-226; Shaw et al., 2004, Cancer Cell, Vol. 6(1) : 91-99). Thus, it can be hypothesized that AMPK inhibition induced by FuFA-F2 observed in this study is responsible for the stimulation of the mTOR pathway in C2C12 myotubes. It was next investigated protein degradation. Autophagy is a major intracellular degradation process that is essential for the clearance of altered proteins and organelles. In addition, autophagy has been shown to be critical for skeletal muscle homeostasis (Masiero et al., 2009, Cell Metabolims, Vol. 10(6): 507-515). This process involves the formation of double-membraned structures known as autophagosomes. LC3-II is a standard marker for autophagosomes, so we assessed autophagy by measuring the LC3-II/LC3-I ratio. It was found that FuFA-F2 decreased significantly this ratio whatever the dose indicating that this fatty acid inhibited the autophagy process in C2C12 myotubes. It was also found that FuFA-F2 decreased AMPK activation, a regulator of autophagy initiation (Hardie et al., 2012, Nat Rev Mol Cell Bio, Vol. 13(4) : 251-262). Thus, FuFA-F2 increases the anabolism of C2C12 myotubes by increasing protein synthesis on the one hand and inhibiting autophagy on the other. In addition, it was found herein that this stimulation of anabolism induced by FuFA-F2 was also accompanied by a change in the metabolic and contractile characteristics of muscle fibers for the intermediate dose of 10 µM. By stimulating the mitochondrial respiratory chain and inducing the expression of slow myosin heavy chain, FuFA-F2 supplementation could promote the utilization of glucose by the muscle. In a pathological context, this may delay or limit insulin resistance.

To investigate *in vivo* the potential impact of FuFA-F2 on muscle characteristics, C57Bl6 mice were supplemented by oral gavage with vehicle or FuFA-F2. After only 3 weeks of gavage in C57BL6 mice it was observed herein that FuFA-F2 increased in muscle mass and promoted a more oxidative metabolism. However, the mechanisms of action and metabolic pathways of FuFA-F2 at the muscle and cellular level remain to be explored.

### Example 3 : In vivo effects of FuFA-F2 incorporated in a High Fat and High Sucrose (HFHS) diet on muscle mass

### A. Materials and Methods

### Animals and Ethics statement

Forty six-weeks old male C57BL/6J mice (Charles River, L'Arbresle, France), weighing about 21 g, were housed (5 per cage) under conditions of constant temperature (20-22°C), humidity (45-50%) and a standard dark cycle (20.00-08.00 hours). The mice were randomized, according to their initial weight, into four groups of ten animals and fed for 12 weeks with one of the four following semi-purified diets: 1) control diet, 2) HFHS diet, 3) HFHS + FuFA-F2 (0.048%, 40mg/kg body weight/day), 4) HFHS + FuFA-F2 (0.127%, 105mg/kg body weight/day). In this example, "FuFA-F2" is the triacylglycerol form of FuFA-F2.

The control diet contains 5% lipids as a mixture of rapeseed oil, high oleic sunflower oil, sunflower oil and linseed oil (oil mixture of Carrefour), the high fat high sucrose (HFHS) diets contain 25% lipids (5% of a mixture of rapeseed oil, high oleic sunflower oil, sunflower oil and linseed oil and 20% of lard) and 30% sucrose. The detailed composition of the diets is given in Table 1 at the end of the present description.

The FuFA-F2 was incorporated into the diet after dissolution in the combined table oil (Isio4 oil of Carrefour) and the final content was set at 0.048% and 0.127% respectively for HFHS+FuFA-F2 (0.048%) and HFHS+FuFA-F2 (0.127%) groups. For FuFA-F2 (0.048% w/w), 2.39 g of FuFA-1 was dissolved in 247.61 g of Isio-4^{®} commercial oil to reach a final composition of 250 g. For FuFA-2 (0.127% w/w), 6.30 g of FuFA-2 was dissolved in 243.70 g of Isio-4^{®} commercial oil to reach a final composition of 250 g.

Throughout the study, mice were given free access to food and tap water. Mice body weight was followed weekly and food consumption was determined every two days (during the week) or three days (the week-end). Our institution guidelines for the care and use of laboratory animals were observed and all experimental procedures were approved by the local ethical committee in Montpellier, France (Reference APAFIS#31900-2021060417205673 v2).

### Body composition

After cervical dislocation of mice, muscle quadriceps were removed and weight.

### Statistical analysis

Results were expressed as means ± SD. Groups were tested for the effects of dietary intake by a one-way ANOVA test, followed up by a Fisher's Least Significant Difference test. The limit of statistical significance was set at p<0.05. The means with different letters were significantly different. Statistical analyses were performed using the StatView program (SAS Institute, Cary, NC, USA).

### B. Results

The results are depicted in Figure 8.

We observed a significant increase in quadriceps muscle mass in mice receiving a HFHS diet for 12 weeks (p<0.001) by comparison to control. Furthermore, our results showed that enrichment of the HFHS diet with FuFA-F2 at any dose increased quadriceps muscle mass compared to HFHS alone and that the highest dose of FuFA-F2 (105mg/kg body weight/day) significantly increased quadriceps muscle mass by comparison to the lowest dose of FuFA-F2 (40mg/kg body weight/day).

### Conclusions

In conclusion, the inventors have shown for the first time that supplementation in FuFA-F2 increases protein content in C2C12 myotubes . This anabolic effect is explained by the fact that treatment of myotubes with FuFA-F2: i) decreases autophagy by inhibiting autophagosomes formation as attested by the decrease of LC3II/LC3I ratio; ii) increases protein synthesis as shown by the stimulation of mTOR phosphorylation. In addition, the inventors have found that FuFA-F2 modulates the metabolic and contractile characteristics of C2C12 myotubes.

In addition, the inventors have found that oral gavage with FuFA-F2 for 3 weeks in C57Bl6 mice increased muscle mass and promoted more oxidative muscle metabolism in mice. Altogether, these data demonstrated that FuFA-F2 extracted from *Hevea brasiliensis* latex stimulates muscle anabolism both *in vitro* and *in vivo,* and promotes a more oxidative metabolism. These results highlight that FuFA-F2 supplementation could have health benefits by promoting skeletal muscle anabolism.

Further, the examples also show that feeding subjects with various amounts FuFA-containing compounds specified in the present disclosure cause an increase of muscle mass, including in a dose-dependent manner.

Such effect could be particularly beneficial 1) in elderly people suffering from sarcopenia, 2) during prolonged immobilization responsible for muscle wasting, 3) in patients with cancer cachexia. Finally, supplementation with a furan fatty acid or a furan fatty acid-containing compound according to the present disclosure can also be of interest to improve the performance of athletes.

Thus, it is shown herein that furan fatty acids or a furan fatty acid-containing compounds according to the present disclosure represent new biosourced molecule derived from the latex of *Hevea brasiliensis,* currently used for natural rubber production, produced in an environmentally sustainable way, with a strong therapeutic potential.

**Table 1 : Detailed constitution of the nutritional compositions used in Example 3**

| **Ingredients** | **Control Diet** | **HFHS Diet** | **HFHS** + **FuFA-2 (0.048%)** | **HFHS** + **FuFA-2 (0.127%)** |
|---|---|---|---|---|
| | **g/kg** | **g/kg** | **g/kg** | **g/kg** |
| **Casein** | 200 | 200 | 200 | 200 |
| **Maize Starch** | 450 | 115 | 115 | 115 |
| **Maltodextrin** | 150 | 35 | 35 | 35 |
| **Saccharose** | 50 | 300 | 300 | 300 |
| **Commercial Isio-4^{®} oil mixture** | 50 | 50 | 49.52 | 48.73 |
| **FuFA-F2** | 0 | 0 | 0.48 | 1.27 |
| **Lard** | 0 | 200 | 200 | 200 |
| **Mineral mixture (AIN-93M)** | 35 | 35 | 35 | 35 |
| **Vitamins (AIN-93M)** | 10 | 10 | 10 | 10 |
| **L-Cystine** | 2.5 | 2.5 | 2.5 | 2.5 |
| **Choline bitartrate** | 2.5 | 2.5 | 2.5 | 2.5 |
| **TOTAL** | 1000 | 1000 | 1000 | 1000 |
| **Form** | granulates | granulates | granulates | granulates |

## Claims

1. A furan fatty acid, or a furan fatty acid-containing compound, for its use for preventing and/or treating a loss in muscle mass in a human or animal subject.

2. A furan fatty acid, or a furan fatty acid-containing compound, for its use according to claim 1, wherein the furan fatty acid or the furan fatty acid-containing compound is of formula (I) : 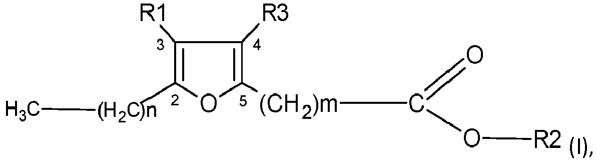 wherein
(i) m is an integer ranging from 1 to 12 ,
(ii) n is an integer ranging from 1 to 12,
(iii) R1and R3 represent, independently of each other, a hydrogen atom, a methyl group or a carboxyl group, and
(iv) R2 means a hydrogen atom, a (C1-C22) alkyl group, a (C2-C22) alkenyl group, a (C2-C22) alkynyl group or a group of formula (A1) or (A2) : 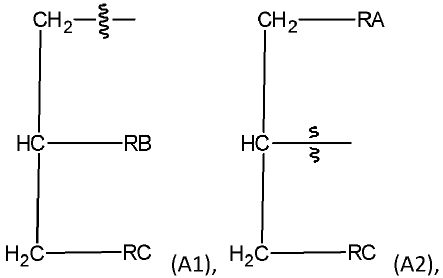 wherein 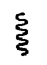
- symbol« » in formula (A1) means the attachment site between the CH₂ group thereof and the oxygen atom of the carboxyl group of the compound of formula (I), 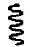
- symbol« » in formula (A2) means the attachment site between the CH group thereof and the oxygen atom of the carboxyl group of the compound of formula (I), and
- RA, RB and RC represent, independently of each other,
- a hydroxyl group, or
- -O-C(=O)-R4 , wherein R4 means :
• a group of formula (III) : 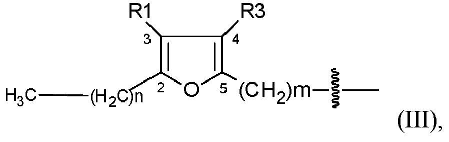 (III), wherein R1, R3, 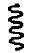 m and n are as defined for compound of formula (I) and symbol« » in formula (III) means the attachment site between the (CH₂)m thereof and the carbonyl group of the -O-C(=O)-R4, or
• a (C6-C22) linear or branched alkyl group, a (C6-C22) linear or branched alkenyl group or a (C6-C22) linear or branched alkynyl group.

3. A furan fatty acid or furan fatty acid-containing compound for its use according to claim 2, wherein
- each of R1, R3, m and n, are as defined in claim 2,
- R2 means a group of formula (A1) wherein RB and RC are as defined in claim 2 with at least one of RB and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH2)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂, or
- R2 means a group of formula (A2) wherein RA and RC are as defined in claim 2 with at least one of RA and RC meaning -O-C(=O)-R4 wherein R4 is selected in the group consisting of (i) CH₃(CH₂)₄-, (ii) CH₃(CH₂)₆-, (iii) CH₃(CH₂)₈-, (iv) CH₃(CH₂)₁₀-, (v) CH₃(CH₂)₁₂-, (vi) CH₃(CH₂)₁₄-, (vii) CH₃(CH₂)₁₆-, (viii) CH₃(CH2)₁₈-, (ix) CH₃(CH₂)₂₀-, (x) CH₃(CH₂)₂₂-, (xi) CH₃(CH₂)₂₄-, (xii) CH₃(CH₂)₃-CH=CH(CH₂)₇-, (xiii) CH₃(CH₂)₅-CH=CH-(CH₂)₇-, (xiv) CH₃(CH₂)₈-CH=CH-(CH₂)₄-, (xv) CH₃(CH₂)₇-CH=CH-(CH₂)₇-, (xvi) CH₃(CH₂)₇-CH=CH-(CH₂)₈-, (xvii) CH₃(CH₂)₅-CH=CH-(CH₂)₉-, (xviii) CH₃(CH₂)₄-CH=CH-CH₂-CH=CH-CH₂)₇-, (xix) CH₃-CH₂-CH=CH-CH₂-CH=CH-CH₂-CH=CH-(CH₂)₇-, (xx) CH₃(CH₂)₄-CH=CH-CH₂-CH=CH-CH₂-CH=CH-CH₂-CH=CH-(CH₂)₃-, (xxi) CH₃-CH₂-CH=CH-CH₂-CH=CH-CH₂-CH=CH-CH₂-CH=CH-CH₂-CH=CH-(CH₂)₃- and (xxii) CH₃-CH₂-CH=CH-CH₂-CH=CH-CH₂-CH=CH-CH₂-CH=CH-CH₂-CH=CH-CH₂₋CH=CH-(CH₂)₂.

4. A furan fatty acid for its use according to claim 2, which is selected from the furan fatty acids wherein,
- n is 2, m is 8, R1 means a methyl group, R2 means a hydrogen atom and R3 means a methyl group, or
- n is 4, m is 8, R1 means a hydrogen atom, R2 means a hydrogen atom and R3 means a methyl group, or
- n is 4, m is 8, R1 means a methyl group, R2 means a hydrogen atom and R3 means a methyl group, or
- n is 2, m is 10, R1 means a methyl group, R2 means a hydrogen atom and R3 means a methyl group, or
- n is 4, m is 10, R1 means a hydrogen atom, R2 means a hydrogen atom and R3 means a methyl group, or
- n is 4, m is 10, R1 means a methyl group, R2 means a hydrogen atom and R3 means a methyl group, or
- n is 4, m is 12, R1 means a hydrogen atom, R2 means a hydrogen atom and R3 means a methyl group , or
- n is 4, m is 12, R1 means a methyl group, R2 means a hydrogen atom and R3 means a methyl group.

5. A furan fatty acid for its use according to claim 2, wherein the furan fatty acid is of formula (I) wherein n is 4, m is 8, R1 means a hydrogen atom, R2 means a hydrogen atom and R3 means a methyl group.

6. A furan fatty acid-containing compound for its use according to any one of claims 1 to 3, which is of formula (II) below 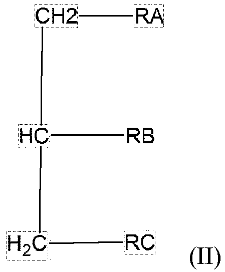 wherein groups RA, RB and RC represent, independently of each other, (i) a hydroxyl group, (ii) -O-C(=O)-R4 , wherein R4 means a group of formula (III) as defined in claim 2 or (iii) -O-C(=O)-R4 , wherein R4 means a (C6-C22) linear or branched, alkyl chain, a (C6-C22) linear or branched alkenyl group or a (C6-C22) linear or branched alkynyl group, provided that at least one of RA, RB and RC represents -O-C(=O)-R4 , wherein R4 means a group of formula (III) as defined in claim 2.

7. A furan fatty acid-containing compound of formula (II) according to claim 5, wherein RA, RB and RC are identical and each means -O-C(=O)-R4 , wherein R4 means a group of formula (III) as defined in claim 2 wherein n is 4, m is 8, R1 means a hydrogen atom and R3 means a methyl group.

8. A furan fatty acid, or a furan fatty acid-containing compound, for its use according to any one of claims 1 to 7, wherein the subject is a human subject or an animal subject.

9. A furan fatty acid, or a furan fatty acid-containing compound, for its use according to any one of claims 1 to 8, wherein the human or animal subject is selected among elderly subjects affected by sarcopenia, overweight and obese subjects affected by sarcopenic obesity and subjected to a food regime, subjects affected by cachexia, notably a cachexia linked to cancer, subjects affected by an inflammatory bowel disease or a chronic obstructive pulmonary disease, immobilized subjects, subjects with severe burns and affected with septic or viral infections such as with a SARS-CoV-2 virus, subjects having a heart failure or a respiratory failure, subjects having cirrhosis, subjects affected with a rheumatoid arthritis,, convalescent subjects, immobilized subjects, and subjects with bowel resections or intestinal malabsorptions.

10. A furan fatty acid, or a furan fatty acid-containing compound, for its use according to any one of claims 1 to 8, which is comprised in an oral composition, and more particularly in an oral composition selected from the group consisting of a food product, a beverage, a pharmaceutical, a nutraceutical, a food additive, a dietary supplement, a dairy product and a living biotherapy product (LBPs).

11. A non-therapeutic use of a furan fatty acid, or of a furan fatty acid-containing compound as defined in any one of claims 1 to 7, for maintaining or increasing muscle mass in a subject in need thereof, in particular in a subject selected from a malnourished subject, an elderly subject, in particular a malnourished elderly subject, and a subject engaged in strenuous exercise.

12. The non-therapeutic use according to claim 11, wherein the furan fatty acid, or the furan fatty acid-containing compound is of formula (I) or (II) as defined in any one of claims 2 to 6.
